# EUROPEAN PATENT SPECIFICATION

(11) **EP 0 558 677 B1**
(45) Date of publication and mention of the grant of the patent: **16.08.2001**
(21) Application number: 92902511.2
(22) Date of filing: 19.11.1991
(51) Int. Cl.: C07D 277/68, C07D 235/26, C07D 209/34, C07C 233/43

(54) **BIOLOGICALLY ACTIVE AMINES**
BIOLOGISCH AKTIVE AMINE
AMINES BIOLOGIQUEMENT ACTIVES

(30) Priority: 20.11.1990 GB 9025176; 20.11.1990 GB 9025178; 20.11.1990 GB 9025180
(43) Date of publication of application: 08.09.1993
(73) Proprietor: AstraZeneca AB, 151 85 Södertälje (SE)
(72) Inventor: BROWN, Roger Charles, Loughborough, Leicestershire LE11 0QN (GB); DIXON, John, Nr Melton Mowbray, Leicesters. LE14 2ETT (GB); INCE, Francis, Loughborough, Leicestershire LE11 3SP (GB); CHESHIRE, David Rayuls, Beeston, Nottinghamshire NG9 1DJ (GB); BONNERT, Roger Victor, Loughborough, Leicestershire LE12 5SE (GB)
(74) Representative: As Sivborg, Susanne Birgitta
(86) International application number: GB9102042
(87) International publication number: WO9208708

(56) References cited:
- EP-A- 0 046 666
- EP-A- 0 113 964
- EP-A- 0 174 811
- EP-A- 0 175 525
- EP-A- 0 178 919
- EP-A- 0 180 994
- EP-A- 0 304 789
- J. Med. Chem., Vol. 30, 1987 Joseph Weinstock et al: "Synthesis and Evaluation of Non-Catechol D-1 and D-2 Dopamine Receptor Agonists: Benzimidazol-2-one, Benzoxazol -2-one, and the Highly Potent Benzothiazol-2-one 7- ",

## Description

This invention relates to novel compounds, processes for their preparation, compositions containing them and methods of treatment involving their use.

EP-A-174 811 describes certain 1,3 benzthiazol-2(3H)-ones and 1,3-benzoxazol-2(3H)-ones having an aminoethyl substituent in the benz ring which are said to have dopamine D₂-agonist activity. In addition, certain benzimidazol derivatives having an aminoethyl substituent in the benz ring are known to be dopamine D₂-agonists from EP-A-175 525 and EP-A-304 789.

According to the invention there are provided compounds of formula I,

Ar-CH₂-CH₂-NH-CR¹R²-X-Y

in which
Ar represents a group 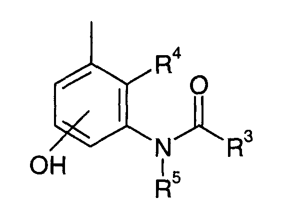
X represents a C₁₋₁₂ alkylene chain interrupted or terminated by one or more groups selected from -S-, -SO-, -SO₂-, -O-, CR⁶R⁷, phenylmethyne, -NH-, -CONH-, -NHCO- and -NHCONH-,
Y represents an aryl group of five or six atoms in a single ring system, which may contain from 1 to 3 heteroatoms selected from N, O and S, which single ring system may be optionally substituted to form a multiple fused ring system of up to 10 atoms, the aryl group being optionally substituted by one or more groups selected from -OH, halogen, C₁₋₆ alkyl, C₁₋₆ alkoxy, =O, -NH₂, or NO₂; or a C₃₋₁₂ cycloalkyl group which may contain from 1 to 3 heteroatoms selected from N, O and S, optionally substituted by one or more groups selected from -OH, halogen, C₁₋₆ alkyl, C₁₋₆ alkoxy, =O, -NH₂, or NO₂,
R¹, R², R⁵, R⁶, R⁷ and R⁸ each independently represent hydrogen or alkyl C₁₋₆, and
R³ and R⁴ represent hydrogen, or R³ and R⁴ together form a group -S-, -NR⁸- or -CH₂-,
and pharmaceutically acceptable derivatives thereof.

According to the invention we also provide a process for the production of a compound of formula I, or a pharmaceutically acceptable derivative thereof, which comprises
a) alkylation of a compound of formula II, or a derivative thereof,

   Ar-CH₂CH₂-NH₂ II

   in which Ar is as defined above,
   with an alkylating agent of formula III,

   L-CR¹R²-X-Y III

   in which R¹, R², X and Y are as defined above and L represents a leaving group,
b) alkylation of a compound of formula II, as defined above, with a compound of formula IV,

   O=CR¹-X-Y IV

   in which R¹, X and Y are as defined above,
   in the presence of a reducing agent,
c) selective reduction of a compound of formula V, 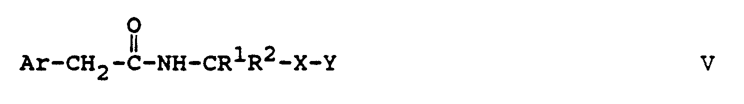 in which Ar, X, Y, R¹ and R² are as defined above, or
   for the production of compounds of formula I in which R¹ and R² represent hydrogen selective reduction of a compound of formula Va, 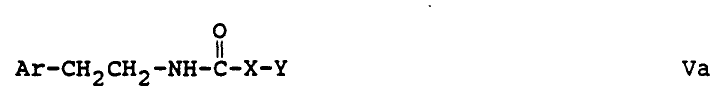 in which Ar, X and Y are as defined above,
d) removal of a protecting group from a corresponding protected compound of formula I in which one or more of the functional groups is protected,
   and where desired or necessary converting the resulting compound of formula I to a pharmaceutically acceptable derivative thereof, or vice versa.

In process a) leaving groups which L may represent include halogen, such as chlorine, bromine and iodine, and alkyl or arylsulphonyloxy groups such as methanesulphonyloxy or p-toluenesulphonyloxy. The reaction is preferably carried out in the presence of a base, e.g. an inorganic base such as sodium or potassium carbonate, or an organic base such as triethylamine, N,N-diisopropylethylamine or pyridine. The reaction is conveniently performed in a solvent such as an ether, e.g. tetrahydrofuran or dioxan, a ketone, e.g. butanone or methyl isobutyl ketone, a substituted amide, e.g. dimethylformamide, or a chlorinated hydrocarbon, e.g. chloroform, at a temperature of between ambient temperature and the reflux temperature of the solvent.

The alkylating agent of formula III may be prepared from the corresponding alcohol (i.e. the compound in which L represents OH) by known methods. For example, the alcohol may be reacted with a halogenating agent to yield the compound of formula III in which L represents a halogen atom. Suitable halogenating agents include, for example, triphenylphosphine-tetrahalogenomethane adduct (conveniently formed in situ, e.g. by the reaction of triphenylphosphine and carbontetrabromide). The reaction may take place in the presence of a solvent such as acetonitrile, or a chlorinated hydrocarbon, e.g. dichloromethane, at a temperature in the range of 0-30°C.

In process b) suitable reducing agents include hydrogen in the presence of a catalyst such as platinum, platinum oxide, palladium, palladium oxide, Raney nickel or rhodium, on a support such as charcoal, using an alcohol, e.g. ethanol, or an ester, e.g. ethyl acetate, or an ether, e.g. tetrahydrofuran, or water, as reaction solvent, or a mixture of solvents, at normal or elevated temperature and pressure. Alternatively the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols, such as methanol or ethanol, or ethers, such as diethyl ether or t-butyl methyl ether, or tetrahydrofuran.

Alkylation using the compound of formula IV may give rise to an intermediate imine, reduction of which under the conditions described yields the compound of formula I.

The compounds of formulae II and IV and the alcohols corresponding to formula III are either known or may be prepared by known techniques.

In process c) the reaction may be carried out using conventional reduction techniques. The reducing agent may be electrophilic, e.g. diborane, or nucleophilic, e.g. a complex metal hydride such as lithium aluminium hydride or sodium bis(2-methoxyethoxy)aluminium hydride. The solvent is preferably inert to the reaction conditions. Aprotic solvents are preferred, e.g. tetrahydrofuran, diethyl ether, or 1,2-dimethoxyethane. The reaction may be carried out at a temperature of from about 0 to 100°C.

The compounds of formula V and Va may be prepared by coupling of an amine and an acid or acid chloride by conventional means. For example, the coupling may be performed in the presence of dicyclohexylcarbodiimide using the method of Sheehan and Hess, J. Am. Chem. Soc., 1955, 77, 1067; or 1,1-dicarbonyldiimidazole as described by Staab, Angew. Chem. Int. Ed. Engl., 1962, 1, 351. The amines required for the coupling reaction are either known or may be prepared by conventional methods, for example, compounds in which R³ and R⁴ together represent -S- may be prepared as described in J. Med. Chem., 1987, 30, 1166.

The intermediates of formula Va are novel, thus according to a further aspect of the invention there are provided compounds of formula Va, 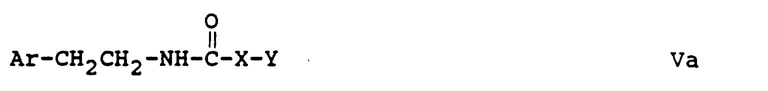 in which Ar, X and Y are as defined above.

Further preparative details for the compounds of formula I are given in the Examples.

In the above processes it may be necessary for any functional groups, e.g. hydroxy or amino groups, present in the starting materials to be protected, thus process d) may involve the removal of one or more protecting groups. Suitable protecting groups and methods for their removal are, for example, those described in "Protective Groups in Organic Chemistry" by Theodora Greene, John Wiley and Sons Inc., 1981. Hydroxy groups may, for example, be protected by arylmethyl groups such as phenylmethyl, diphenylmethyl or triphenylmethyl, or as tetrahydropyranyl derivatives.

Suitable amino protecting groups include arylmethyl groups such as benzyl, (R,S)-α-phenylethyl, diphenylmethyl or triphenylmethyl, and acyl groups such as acetyl, trichloroacetyl or trifluoroacetyl. Conventional methods of deprotection may be used. Arylmethyl groups may, for example, be removed by hydrogenolysis in the presence of a metal catalyst e.g. palladium on charcoal. Tetrahydropyranyl groups may be cleaved by hydrolysis under acidic conditions. Acyl groups may be removed by hydrolysis with a base such as sodium hydroxide or potassium carbonate, or a group such as trichloroacetyl may be removed by reduction with, for example, zinc and acetic acid.

Pharmaceutically acceptable derivatives of the compound of formula I include pharmaceutically acceptable salts, esters and amides thereof.

Suitable pharmaceutically acceptable salts of the compounds of formula I include acid addition salts derived from inorganic and organic acids, such as hydrochlorides, hydrobromides, sulphates, phosphates, maleates, tartrates, citrates, benzoates, 4-methoxybenzoates, 2- or 4-hydroxybenzoates, 4-chlorobenzoates, benzenesulphonates, p-toluenesulphonates, naphthalenesulphonates, methanesulphonates, sulphamates, ascorbates, salicylates, acetates, diphenylacetates, triphenylacetates, adipates, fumarates, succinates, lactates, glutarates, gluconates, hydroxy-naphthalenecarboxylates, e.g. 1-hydroxy or 3-hydroxy-2-naphthalenecarboxylates, or oleates. The compounds may also form salts with suitable bases. Examples of such salts include alkali metal, e.g. sodium and potassium, and alkaline earth metal, e.g. calcium and magnesium, salts. The compound of formula I may be obtained in the form of a salt, conveniently a pharmaceutically acceptable salt. Where desired, such salts may be converted to the free bases using conventional methods. Pharmaceutically acceptable salts may be prepared by reacting the compound of formula I with an appropriate acid or base in the presence of a suitable solvent.

Suitable pharmaceutically acceptable esters of the compounds of formula I include lower alkyl esters, e.g. ethyl ester. The esters may be made by conventional techniques, e.g. esterification or transesterification.

Suitable amides include unsubstituted or mono- or di-substituted alkyl C₁₋₆ or phenyl amides, and may be made by conventional techniques, e.g. reaction of an ester of the corresponding acid with ammonia or an appropriate amine.

The compounds of formula I may exhibit tautomerism, they may also contain one or more asymmetric carbon atoms and may therefore exhibit optical and/or diastereoisomerism. Diastereoisomers may be separated using conventional techniques, e.g. chromatography or fractional crystallisation. The various optical isomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation.

By the term alkyl we mean straight, branched or cyclic saturated or unsaturated alkyl groups.

The term cycloalkyl includes C₃₋₁₂ cyclic groups. The cycloalkyl group may contain from 1 to 3 heteroatoms selected from N, O and S. The cycloalkyl group may be unsubstituted or substituted, for example, by one or more groups selected from -OH, halogen, alkyl C₁₋₆, alkoxy C₁₋₆, =O, -NH₂ or NO₂. Provided that such substituents do not interfere with the efficacy of the compound. Particular cycloalkyl groups which may be mentioned include C₅ and C₆ cycloalkyl groups, e.g. cyclohexane.

The term aryl includes aromatic radicals having five or six atoms in a single ring system, such groups may contain from 1 to 3 heteroatoms selected from N, O and S. The single ring system may be substituted to form a multiple fused ring system containing up to 10 atoms, for example, naphthyl, indenyl or benzothiazolyl. The aryl group may be unsubstituted or substituted, for example, by one or more groups selected from -OH, halogen, alkyl C₁₋₆, alkoxy C₁₋₆, =O, -NH₂ or NO₂. Provided that such substituents do not interfere with the efficacy of the compound. Particular aryl groups which may be mentioned include 5 and 6-membered carbocyclic and heterocyclic aryl groups, for example, furanyl, pyridinyl and thienyl. However, we prefer compounds of formula I in which Y represents phenyl.

Halogens with which Y may be substituted include bromine, chlorine and fluorine.

Where the group X contains more than one group selected from -S-, -SO-, -SO₂-, -O-, CR⁶R⁷, phenylmethyne, -NH-, -CONH-, -NHCO- and -NHCONH-, then those groups may be the same or different.

We prefer compounds in which X is interrupted or terminated by at least one group, and more preferably one to three groups, e.g. one or two groups, selected from -S-, -SO-, -SO₂-, -O-, CR⁶R⁷, phenylmethyne, -NH-, -CONH-, -NHCO- and -NHCONH-

Specific groups which X may represent include the following:
-(CH₂)₂SO₂(CH₂)₂O(CH₂)₂-
-CH₂SO₂(CH₂)₃O(CH₂)₂-
-(CH₂)₅O-
-(CH₂)₅OCH₂-
-(CH₂)₅O(CH₂)₂-
-(CH₂)₅O(CH₂)₃-
-(CH₂)₅O(CH₂)₄-
-(CH₂)₃O(CH₂)₂-
-(CH₂)₂SO₂(CH₂)₂NH(CH₂)₂-
-(CH₂)₄CONH(CH₂)₂-
-CH₂SO₂(CH₂)₂CONH(CH₂)₂-
-(CH₂)₅NHCONH(CH₂)₂-
-CHMe(CH₂)₄O(CH₂)₂-
-CMe₂(CH₂)₄O(CH₂)₂-

We prefer compounds of formula I in which R₃ and R₄ together represent the group -S-.

We prefer Ar groups in which the -OH is positioned ortho to the NH group.

When R¹ and R² are other than hydrogen, alkyl groups which they may represent include alkyl C₁₋₃, for example, methyl.

We prefer compounds of formula I in which R⁵ represents hydrogen.

The compounds of formula I are useful in that they exhibit pharmacological activity in animals. In particular the compounds are β₂-adrenoreceptor agonists. The activity may be demonstrated in the isolated trachea of the guinea pig, as described by T.P. Kenakin, Br. J. Pharmacol., 1980, 71, 407. The compounds are also dopamine DA₂-agonists. The binding affinities of the test compounds for the DA₂ binding sites in bovine pituitary membranes may be determined from the displacement of [³H]-N-n-propylnorapomorphine and of [³H]-spiperone in the absence or presence of nonhydrolysable GTP analogue respectively, D.R. Sibley, A. DeLean and I. Creese, Anterior Pituitary Dopamine Receptors, Demonstration of Interconvertible High and Low Affinity Sites of the D-2 Dopamine Receptor, J. Biol. Chem., 1982, 257(11), 6351-6361. The DA₂ activity may also be demonstrated in a functional screen, the rabbit isolated ear artery, Brown and O'Connor, Br. J. Pharmacol., 1981, 73, 189P. The compounds also show advantageous DA₂:β₂ activity ratios.

The compounds of formula I are indicated for use in the treatment of the range of conditions known as reversible obstructive airways disease, e.g. asthma and bronchitis.

According to another aspect of the invention there is therefore provided a method of treatment or prophylaxis of reversible obstructive airways disease, which method comprises administering a therapeutically effective quantity of a compound of formula I, or a pharmaceutically acceptable derivative thereof, to a patient suffering from or susceptible to such a condition.

The compounds of formula I are also indicated for use in the treatment of various other conditions, e.g. inflammatory and allergic skin disorders, congestive heart failure and glaucoma.

For the above mentioned uses the doses administered will, of course, vary with compound employed, the mode of administration and the treatment desired. However, in general, satisfactory results are obtained when the compound is administered at a daily dosage of from about 0.1 mg to about 20 mg per kg of animal body weight, preferably given in divided doses 1 to 4 times a day or in sustained release form. For man the total daily dose is in the range of from 7.0 mg to 1,400 mg and unit dosage forms suitable for oral administration comprise from 2.0 mg to 1,400 mg of the compound admixed with a solid or liquid pharmaceutical diluent or carrier.

The compounds of formula I may be used on their own or in the form of appropriate medicinal preparations for enteral, parenteral or topical administration.

According to the invention there is also provided a pharmaceutical composition comprising preferably less than 80% and more preferably less than 50% by weight of a compound of formula I, or a pharmaceutically acceptable derivative thereof, in admixture with a pharmaceutically acceptable adjuvant, diluent or carrier.

Examples of such adjuvants, diluents and carriers are
for tablets and dragees - lactose, starch, talc, stearic acid;
for capsules - tartaric acid or lactose;
for injectable solutions - water, alcohols, glycerin, vegetable oils;
for suppositories - natural or hardened oils or waxes.
Compositions in a form suitable for oesophageal administration include tablets, capsules and dragees;
compositions in a form suitable for administration to the lung include aerosols, particularly pressurised aerosols;
compositions in a form suitable for administration to the skin include creams, e.g. oil-in-water emulsions or water-in-oil emulsions;
compositions in a form suitable for administration to the eye include drops and ointments.

The compounds of formula I have the advantage that they are less toxic, more efficacious, are longer acting, have a broader range of activity, are more potent, produce fewer side effects, are more easily absorbed or have other useful pharmacological properties, than compounds of similar structure.

The invention is illustrated, but in no way limited, by the following Examples, in which temperatures are in degrees Celsius. The reactions were performed under an inert atmosphere of either nitrogen or argon.

### Example 1

### 7-[2-[6-[2-(2,3-Dihydro-1H-indenyloxy)]hexyl]amino-ethyl]-4-hydroxy-1,3-benzthiazol-2(3H)-one hydrochloride

### a) N-[2-(4-Hydroxy-2-oxo-3H-1,3-benzothiazol-7-yl)ethyl]-6-[2-(2,3-dihydro-1H-indenyloxy)]hexanamide

To a stirred solution of 7-(2-aminoethyl)-4-hydroxy-1,3-benzothiazol-2(3H)-one hydrobromide (1.50 g) and 6-[2-(2,3-dihydro-lH-indenyloxy)]hexanoic acid (1.41 g) in dimethylformamide (25 ml) was added triethylamine (0.72 ml), 1-hydroxybenzotriazole hydrate (1.905 g) and dicyclohexylcarbodiimide (1.38 g). The mixture was stirred for 40 hours at room temperature. Glacial acetic acid (0.4 ml) was added and stirring continued for 15 min. The dimethylformamide was removed under reduced pressure, the residue slurried with ethyl acetate (50 ml) and the suspended dicyclohexylurea removed by filtration. The filtrate was washed with saturated aqueous sodium bicarbonate and dried (MgSO₄). The solvent was removed under reduced pressure to yield a residue which was purified by column chromatography on silica using 5% ethanol in dichloromethane as eluant, to give the sub-titled compound as a yellow oil.

nmr δ (d₆DMSO) 1.22 (m, 2H), 1.45 (brm, 4H), 2.00 (t, 2H), 2.58 (t, 2H), 2.80 (d, 1H), 2.85 (d, 1H), 3.06 (d, 1H), 3.10 (d, 1H), 3.22 (q, 2H), 3.4 (m, 2H+H₂O), 4.26 (m, 1H), 6.70 (d, 1H), 6.80 (d, 1H), 7.1-7.2 (m, 4H), 7.83 (t, 1H), 9.88 (brs, 1H), 11.61 (s, 1H).

### b) 7-[2-[6-[2-(2,3-Dihydro-1H-indenyloxy)]hexyl]amino-ethyl]-4-hydroxy-1,3-benzthiazol-2(3H)-one hydrochloride

Borane-tetrahydrofuran solution (1.0 M in THF, 11 ml) was added dropwise to a stirred solution of the product from step a) (1.39 g) in dry tetrahydrofuran. The mixture was heated under reflux until thin layer chromatography indicated the reaction had gone to completion, about 3 hours. The reaction was cooled and methanol (2.5 ml) added, the mixture was then heated under reflux for a further 15 min. The solvent was removed under reduced pressure and the residue dissolved in methanol (25 ml) to which was added conc. hydrochloric acid (sg. 1.18, 0.55 ml). The solution was heated under reflux for 30 min. Cooling and removal of the methanol under reduced pressure yielded an oily residue which when triturated with ether gave the title compound as an off white solid, which was further purified by reverse phase HPLC using methanol/0.1% aqueous trifluoroacetate as eluant.
mp 220-223°;
Mass Spectrum: FAB 427 ((M+H)⁺);
nmr δ (d₆DMSO) 1.23 (brs, 4H), 1.48 (brt, 2H), 1.59 (brm, 2H), 2.85 (brm, 6H), 3.10 (brm, 4H), 3.42 (t, 2H), 4.26 (m, 1H), 6.76 (d, 1H), 6.86 (d, 1H), 7.12 (m, 2H), 7.20 (m, 2H), 8.80 (brs, 2H), 10.13 (s, 1H), 11.77 (s, 1H);
Analysis: Found C,61.94; H,6.91; N,6.15; S,6.52% C₂₄H₃₀N₂O₃S.HCl requires: C,62.25; H,6.75; N,6.05; S,6.92%.

### Example 2

The following compounds were prepared according to the method of Example 1, from the corresponding intermediate amides:

### a) 4-Hydroxy-7-[2-[6-(2-phenylethoxy)hexyl]aminoethyl]-1,3-benzothiazol-2(3H)-one hydrochloride

### i. N-[2-(4-Hydroxy-2-oxo-3H-1,3-benzothiazol-7-yl)ethyl]-6 - (2-phenylethoxy)hexanamide

mp 139°;
Mass Spectrum: FAB 429 ((M+H)⁺);
nmr δ (d₆DMSO) 1.20 (m, 2H), 1.45 (m, 4H), 2.00 (t, 2H), 2.77 (t, 2H), 3.23 (q, 2H), 3.34 (m, 4H+H₂O), 3.55 (t, 2H), 6.69 (d, 1H), 6.78 (d, 1H), 7.16-7.28 (m, 5H), 7.84 (t, 1H);
Analysis: Found C,64.03; H,6.41; N,6.60; S,6.84%
C₂₃H₂₈N₂O₄S requires: C,64.46; H,6.58; N,6.54; S,7.48%.

### ii. 4-Hydroxy-7-[2-[6-(2-phenylethoxy)hexyl]aminoethyl]-1,3-benzothiazol-2(3H)-one hydrochloride

mp 219-222°C (decomposes);
Mass spectrum: FAB 415 ((M+H)⁺);
nmr δ (d₆DMSO) 1.22-1.37 (m, 4H), 1.47 (t, 2H), 1.54-1.64 (m, 2H), 2.8-2.9 (m, 6H), 3.01-3.12 (brm, 2H), 3.34-3.47 (m, 2H), 3.55 (t, 2H), 6.76 (d, 1H), 6.88 (d, 1H), 7.06-7.32 (m, 5H), 8.87 (brs, 2H), 10.15 (s, 1H), 11.78 (s, 1H);
Analysis: Found C,60.82; H,6.53; N,6.01: S,6.63; Cl,8.34%
C₂₃H₃₀N₂O₃S.HCl requires: C,61.25; H,6.93; N,6.21; S,7.11; Cl,7.86%.

### b) 4-Hydroxy-7-[2-[5-(3-phenylpropoxy)pentyl]aminoethyl]-1,3-benzothiazol-2(3H)-one hydrochloride

### i. N-[2-(4-Hydroxy-2-oxo-3H-1,3-benzothiazol-7-yl)ethyl]-5 - (3-phenylpropoxy)pentanamide

mp 141-142°;
Mass Spectrum: FAB 429 ((M+H)⁺);
nmr δ (CDCl₃) 1.60 (m, 2H), 1.70 (m, 2H), 1.87 (m, 2H), 2.18 (t, 2H), 2.66 (t, 2H), 2.74 (t, 2H), 3.40 (m, 4H), 3.49 (q, 2H), 6.18 (t, 1H), 6.74 (d, 1H), 6.80 (d, 1H), 7.18 (m, 3H), 7.27 (m, 2H), 8.71 (brs, 4H), 10.07 (s, 1H);
Analysis: Found C,64.14; H,6.57; N,6.77; S,7.45%
C₂₃H₂₈N₂O₄S requires: C,64.46; H,6.58; N,6.54; S,7.48%.

### ii. 4-Hydroxy-7-[2-[5-(3-phenylpropoxy)pentyl]aminoethyl]-1,3-benzothiazol-2(3H)-one hydrochloride

mp 211-212° ;
Mass Spectrum: FAB 415 ((M+H)⁺) ;
nmr δ (d₆DMSO) 1.38 (m, 2H), 1.51 (m, 2H), 1.61 (m, 2H), 1.79 (m, 2H), 2.61 (t, 2H), 2.86 (m, 4H), 3.06 (brm, 2H), 3.35 (m, 4H), 6.76 (d, 1H), 6.86 (d, 1H), 7.17 (m, 3H), 7.27 (m, 2H), 8.81 (brs, 2H), 10.13 (5, 1H), 11.77 (s, 1H);
Analysis: Found C,60.83; H,7.02; N,6.21; S,6.94; Cl,8.04%
C₂₃H₃₀N₂O₃S.HCl requires: C,61.25; H,6.93; N,6.21; S,7.11; Cl,7.88%.

### c) 4-Hydroxy-7-[2-[5-[2-(5-methylfuranyl)]pentyl]-aminoethyl]-1,3-benzothiazol-2(3H) -one trifluoroacetate

### i. N-[2-(4-Hydroxy-2-oxo-3H-1,3-benzothiazol-7-yl)ethyl]-5-(5-methylfuran-2-yl)pentanamide

mp 153-154°;
nmr δ (d₆DMSO) 1.47-1.51 (m, 4H), 2.03-2.04 (m, 2H), 2.20 (s, 3H), 2.5 (2H+DMSO), 2.59 (t, 2H), 3.23 (q, 2H), 5.90-5.92 (m, 2H), 6.69 (d, 1H), 6.78 (d, 1H), 7.87 (t, 1H), 9.89 (s, 1H), 11.61 (s, 1H);
Mass Spectrum: FAB 375 ((M+H)⁺).

### ii. 4-Hydroxy-7-[2-[5-[2-(5-methylfuranyl)]pentyl]-aminoethyl]-1,3-benzothiazol-2(3H)-one trifluoroacetate

mp 237-239° (decomposes);
nmr δ (d₆DMSO) 1.30-1.38 (m, 2H), 1.53-1.64 (m, 4H), 2.20 (s, 3H), 2.54 (t, 2H), 2.81 (brt, 2H), 2.94 (t, 2H), 3.10 (t, 2H), 5.93 (m, 2H), 6.75 (d, 1H), 6.86 (d, 1H), 8.56 (brs, 2H), 10.17 (brs, 1H), 11.73 (brs, 1H);
Analysis: Found C,53.27; H,5.46; N,6.00; S,6.66%
C₁₉H₂₄N₂O₃S.CF₃CO₂H.H₂O requires: C,53.04; H,5.51; N,5.89; S,6.80%.

### d) 4-Hydroxy-7-[2-[7-(2-phenylethoxy)heptyl]aminoethyl]-1.3-benzothiazol-2(3H)-one hydrochloride

### i. N-[2-(4-Hydroxy-2-oxo-3H-1,3-benzothiazol-7-yl)ethyl]-7-(2-phenylethoxy)heptanamide

mp 140-142° ;
nmr δ (d₆DMSO) 1.17-1.24 (m, 4H), 1.43-1.48 (m, 4H ), 2.00 (t, 2H), 2.58 (t, 2H), 2.79 (t, 2H), 3.23 (q, 2H), 3.33-3.67 (m, 2H), 3.54 (t, 2H), 6.69 (d, 1H), 6.78 (d, 1H), 7.16-7.29 (m, 5H), 7.48 (t, 1H), 9.89 (s, 1H), 11.61 (s, 1H);

### ii. 4-Hydroxy-7-[2-[7-(2-phenylethoxy)heptyl]aminoethyl]-1,3-benzothiazol-2(3H)-one hydrochloride

mp 225-227°;
nmr δ (d₆DMSO) 1.26 (brs, 6H), 1.43-1.52 (m, 2H), 1.54-1.64 (m, 2H), 2.79 (t, 2H), 2.86-2.92 (m, 4H), 3.01-3.11 (m, 2H), 3.67 (t, 2H+H₂O), 3.56 (t, 2H), 6.78 (d, 1H), 6.87 (d, 1H), 7.16-7.30 (m, 5H), 8.98 (brs, 2H), 10.16 (s, 1H), 11.79 (s, 1H);
Analysis: Found C,61.70; H,7.12; N,6.20; S,6.92%
C₂₄H₃₂N₂O₃S.HCl requires: C,61.98; H,7.15; N,6.02; S,6.89%.

### e) 4-Hydroxy-7-[2-[4-(2-phenylethoxy)butyl]aminoethyl]-1,3-benzothiazol-2(3H)-one hydrochloride

### i. N-[2-(4-Hydroxy-2-oxo-3H-1,3-benzothiazol-7-yl)ethyl]-4-(2-phenylethoxy)butanamide

Mass Spectrum: FAB 401 ((M+H)⁺);
nmr δ (CDCl₃+d₆DMSO) 1.67-1.81 (m, 2H), 2.09 (t, 2H), 2.61 (t, 2H), 2.77 (t, 2H), 3.30-3.36 (m, 4H), 3.53 (t, 2H), 6.40 (brs, 1H), 6.66 (d, 1H), 6.71 (d, 1H), 7.12-7.20 (m, 5H), 8.88 (brs, 1H), 10.49 (brs, 1H).

### ii. 4-Hydroxy-7-[2-[4-(2-phenylethoxy)butyl]aminoethyl]-1,3-benzothiazol-2(3H)-one hydrochloride

mp 204-205°;
nmr δ (d₆DMSO) 1.52-1.57 (m, 2H), 1.61-1.69 (m, 2H), 2.80 (t, 2H), 2.84-2.89 (m, 4H), 3.02 (brm, 2H), 3.40 (t, 2H), 3.57 (t, 2H), 6.78 (d, 1H), 6.87 (d, 1H), 7.15-7.29 (m, 5H), 8.96 (brs, 2H), 10.18 (S, 1H), 11.80 (s, 1H) ;
Analysis: Found C,59.40; H,6.51; N,6.56; S,7.77; Cl,8.00%
C₂₁H₂₆N₂O₃S.HCl requires: C,59.64; H,6.20; N,6.62; S,7.58; Cl,8.30%.

### f) 4-Hydroxy-7-[2-[5-(2-phenylethoxy)pentyl]aminoethyl]-1,3-benzothiazol-2(3H)-one hydrochloride

### i. N-[2-(4-Hydroxy-2-oxo-3H-1,3-benzothiazol-7-yl)ethyl]-5-(2-phenylethoxy)pentanamide

Mass Spectrum: FAB 415 ((M+H)⁺);
nmr δ (CDCl₃) 1.50-1.60 (m, 2H), 1.60-1.70 (m, 2H), 2.18 (brt, 2H), 2.67 (m, 2H), 2.86 (t, 2H), 3.44 (t, 2H) 3.45-3.53 (m, 2H), 3.66 (t, 2H), 6.3 (brs, 1H), 6.65 (d, 1H), 6.72 (d, 1H), 7.19-7.26 (m, 6H), 9.60 (s, 1H).

### ii. 4-Hydroxy-7-[2-[5-(2-phenylethoxy)pentyl]aminoethyl]-1,3-benzothiazol-2(3H)-one hydrochloride

mp 209-210°:
nmr δ (d₆DMSO) 1.28-1.35 (m, 2H), 1.48-1.52 (m, 2H), 1.57-1.68 (m, 2H), 2.80 (t, 2H), 2.85-2.94 (m, 4H), 3.05 (brs, 2H), 3.38 (t, 2H+H₂O), 3.56 (t, 2H), 6.78 (d, 1H), 6.87 (d, 1H), 7.19-7.30 (m, 5H), 8.93 (brs, 2H), 10.16 (s, 1H), 11.79 (s, 1H);
Analysis: Found C,60.09; H,6.45; N,6.36; Cl,9.30%
C₂₂H₂₈N₂O₃S.HCl requires: C,60.47; H,6.69; N,6.41; Cl,8.11%.

### g) 4-Hydroxy-7-[2-[6-(phenylmethoxy)hexyl]aminoethyl]-1,3-benzothiazol-2(3H)-one hydrochloride

### i. N-[2-(4-Hydroxy-2-oxo-3H-1,3-benzothiazol-7-yl)ethyl]-6-(phenylmethoxy)hexanamide

Mass Spectrum: FAB 429 ((M+H)⁺) ;
mp 133-134°;
nmr δ (d₆DMSO) 1.2-1.3 (m, 2H), 1.4-1.55 (m, 4H), 2.01 (t, 2H), 2.57 (t, 2H), 3.2-3.3 (m, 2H), 3.3-3.4 (m, 2H), 4.44 (s, 2H), 6.69 (d, 1H), 6.78 (d, 1H), 7.24-7.37 (m, 5H), 7.85 (t; 1H), 9.91 (s, 1H), 11.61 (s, 1H).

### ii. 4-Hydroxy-7-[2-[6-(phenylmethoxy)hexyl]aminoethyl]-1,3-benzothiazol-2(3H)-one hydrochloride

mp 208-211°;
nmr δ (d₆DMSO) 1.27-1.38 (m, 4H), 1.5-1.65 (m, 4H), 2.81-2.94 (m, 4H), 3.02-3.11 (m, 2H), 3.42 (t, 2H), 4.45 (s, 2H), 6.76 (d, 1H), 6.86 (d, 1H), 7.25-7.38 (m, 5H), 8.81 (brs, 2H), 10.13 (s, 1H), 11.77 (s, 1H);
Analysis: Found C,59.42; H,6.85; N,6.37; S,7.25; Cl,9.44%
C₂₂H₂₈N₂O₃S.HCl requires for 0.17 moles excess HCl: C,59.55; H,6.63; N,6.31; S,7.23; Cl,9.45%.

### h) 4-Hydroxy-7-[2-[4-(4-phenylbutoxy)butyl]aminoethyl]-1,3-benzothiazol-2(3H)-one hydrochloride

### i. N-[2-(4-Hydroxy-2-oxo-3H-1,3-benzothiazol-7-yl)ethyl-4-(4-phenylbutoxy)butanamide

mp 133-135°;
Mass Spectrum: FAB 429 ((M+H)⁺) ;
nmr δ (CDCl₃) 1.06-1.23 (m, 4H), 1.36 (q, 2H), 1.73 (t, 2H), 2.11-2.17 (m, 4H), 2.23 (t, 2H), 2.91-2.94 (m, 4H), 6.25 (d, 1H), 6.31 (d, 1H), 6.66-6.81 (m, 6H), 8.67 (s, 1H), 10.42 (s, 1H).

### ii. 4-Hydroxy-7-[2-[4-(4-phenylbutoxy)butyl]aminoethyl]-1,3-benzothiazol-2(3H)-one hydrochloride

mp 208°;
nmr δ (d₆DMSO) 1.6 (brm, 8H), 2.58 (t, 2H), 2.88 (brm, 4H), 3.05 (t, 2H), 3.45 (m, 4H+H₂O), 6.77 (d, 1H), 6.86 (d, 1H), 7.18 (m, 3H), 7.26 (m, 2H), 8.91 (brs, 2H), 10.16 (s, 1H);
Analysis: Found C,59.21; H,7.08; N,6.17; S,7.22; Cl,8.16%
C₂₃H₃₀N₂O₃S.HCl.0.97H₂O requires: C,58.90; H,7.03; N,5.97; S,6.82; C1,7.60%.

### i) 4-Hydroxy-7-[2-(6-phenoxyhexyl)aminoethyl]-1,3-benzothiazol-2(3H)-one hydrochloride

### i. N-[2-(4-Hydroxy-2-oxo-3H-1,3-benzothiazol-7-yl)ethyl]-6-phenoxyhexanamide

mp 161.5-162.5°;
Mass Spectrum: FAB 407 ((M+H)⁺);
nmr δ (d₆DMSO) 1.30-1.39 (m, 2H), 1.48-1.57 (q, 2H), 1.64-1.72 (m, 2H), 2.03-2.06 (t, 2H), 2.57-2.61 (t, 2H), 3.21-3.26 (m, 2H), 3.90-3.94 (t, 2H), 6.68-6.80 (m, 2H), 6.88-6.91 (m, 3H), 7.24-7.29 (t, 2H), 7.85-7.89 (t, 1H).

### ii. 4-Hydroxy-7-2-(6-phenoxyhexyl)aminoethyl]-1,3-benzothiazol-2(3H)-one hydrochloride

mp 213-214°;
nmr δ (d₆DMSO) 1.26-1.50 (m, 4H), 1.55-1.65 (quin, 2H), 1.65-1.78 (quin, 2H), 2.80-2.85 (t, 2H), 2.90-2.94 (t, 2H), 3.06-3.09 (t, 2H), 3.94-3.97 (t, 2H), 6.74+6.87 (2xd, 2H), 6.90-6.93 (m, 3H), 7.25-7.29 (t, 2H), 8.59 (brs, 2H), 10.10 (brs, 2H);
Analysis: Found C,58.93; H,6.49; N,6.71; S,6.94%
C₂₁H₂₆N₂O₃S.HCl.0.36H₂O requires: C,58.74; H,6.50; N,6.52; S,7.47%.

### j) 4-Hydroxy-7-[2-[6-(3-phenylpropoxy)hexyl]aminoethyl]-1,3-benzothiazol-2(3H)-one oxalate

### i. N-[2-(4-Hydroxy-2-oxo-3H-1,3-benzothiazol-7-yl)ethyl]-6-(3-phenylpropoxy)hexanamide

Mass Spectrum: FAB 443 ((M+H)⁺).

### ii. 4-Hydroxy-7-[2-[6-(3-phenylpropoxy)hexyl]aminoethyl]-1.3-benzothiazol-2(3H)-one oxalate

mp 238-240° (decomposes);
nmr δ (d₆DMSO) 1.31 (brs, 4H), 1.42-1.67 (m, 4H), 1.74-1.81 (quin, 2H), 2.58-2.63 (t, 2H), 2.79-2,85 (brt, 2H), 2.89-2.93 (brt, 2H), 3.05-3.10 (brt, 2H), 3.92-3.55 (t, 4H), 6.74-6.76 (d, 1H), 6.84-6.86 (d, 1H), 7.15-7.29 (m, 5H), 8.4-9.2 (v.brs, not integrated);
Analysis: Found C,59.30; H,6.49; N,5.17%
C₂₄H₃₂N₂O₃S.1.6 C₂H₂O₄ requires: C,59.30; H,6.44; N,5.26%.

### k)4-Hydroxy-7-[2-[2.2-Dimethyl-6-(2-phenylethoxy)hexyl]-aminoethyl]--1.3-benzothiazol-2(3H)-one hydrochloride

### i. N-[2-(4-hydroxy-2-oxo-3H-1,3-benzothiazol-7-yl)ethyl]-2.2-dimethyl-6-(2-phenylethoxy)hexanamide

mp 136-138°;
Mass spectrum: FAB 457 ((M+H)⁺);
nmr δ (d₆DMSO) 1.00 (s, 6H), 1.08-1.12 (m, 2H), 1.34-1.41 (m, 4H), 2.60 (t, 2H), 2.77 (t, 2H), 3.25 (q, 2H), 3.32 (t, 2H), 3.53 (t, 2H), 6.69 (d, 1H), 6.77 (d, 1H), 7.15-7.27 (m, 5H), 7.49 (t, 1H), 9.88 (brs, 1H), 11.61 (brs, 1H):
Analysis: Found C,65.84; H,7.31; N,6.49; S,6.81%
C₂₅H₃₂N₂O₄S requires: C,65.76; H,7.06; N,6.16; S,7.02%.

### ii. 4-Hydroxy-7-[2-[2,2-Dimethyl-6-(2-phenylethoxy)hexyl]-aminoethyl]-1,3-benzothiazol-2(3H)-one hydrochloride

mp 224-225°;
Mass Spectrum: FAB 443 ((M+H)⁺);
nmr δ (d₆DMSO) 0.96 (s, 6H), 1.21-1.30 (m, 4H), 1.45-1.48 (m, 2H), 2.78-2.82 (m, 4H), 2.95-2.99 (m, 2H), 3.02-3.12 (m, 2H), 3.39 (t, 2H), 3.57 (t, 2H), 6.78 (d, 1H), 6.87 (d, 1H), 7.16-7.29 (m, 5H), 8.58 (brs, 2H), 10.17 (s, 1H), 11.78 (s, 1H);
Analysis: Found C,62.62; H,7.51; N,5.91; S,6.67; Cl,8.11%
C₂₅H₃₄N₂O₃S.HCl requires: C,62.27; H,7.36; N,5.85; S,6.69; Cl,7.40%.

### l) 4-Hydroxy-7-[2-[6-(2-phenylethylsulphonyl)hexyl]amino-ethyl]-1,3-benzothiazol-2(3H)-one hydrochloride

### i. N-[2-(4-Hydroxy-2-oxo-1,3-benzothiazol-7-yl)ethyl]-6-(2-phenylethylsulphonyl)hexanamide

mp 174-188°;
Mass Spectrum: FAB 477 ((M+H)⁺);
nmr δ (d₆DMSO) 1.27-1.33 (m, 2H), 1.44-1.52 (m, 2H), 1.61-1.70 (m, 2H), 2.01-2.05 (t, 2H), 2.57-2.61 (t, 2H), 2.98-3.07 (m, 4H), 3.20-3.26 (m, 2H), 3.37-3.43 (m, 2H), 6.69-6.86 (dd, 2H), 7.21-7.31 (m, 5H), 7.86-7.89 (t, 1H), 9.91 (s, 1H), 11.62 (s, 1H).

### ii. 4-Hydroxy-7-[2-[6-(2-phenylethylsulphonyl)hexyl]amino-ethyl]-1,3-benzothiazol-2(3H)-one hydrochloride

mp 210-211°;
Mass Spectrum: FAB 463 ((M+H)⁺) ;
nmr δ (d₆DMSO) 1.35 (brs, 4H), 1.60 (brm, 2H), 1.68 (brm, 2H), 2.83-2.91 (m, 4H), 2.98-3.10 (m, 6H), 3.37-3.42 (m, 2H), 6.75-6.88 (dd, 2H), 7.23-7.26 (m, 1H), 7.31-7.32 (m, 4H), 8.7 (v.brs, 2H);
Analysis: Found C,52.93; H,6.15; N,5.59; S,12.15%
C₂₃H₃₀N₂O₄S.HCl.H₂O requires: C,53.40; H,6.43; N,5.41; S,12.39%.

### m) N-[6-[2-(4-Hydroxy-2-oxo-3H-1,3-benzothiazol-7-yl)-ethylamino]hexyl]-N'-phenyl urea hydrochloride

### i. N-[2-(4-Hydroxy-2-oxo-1,3-benzothiazol-7-yl)ethyl]-6-(phenylaminocarbonylamino)hexanamide

mp 186-188°;
Mass Spectrum: 443 ((M+H)⁺) ;
nmr δ (d₆DMSO) 1.23-1.27 (m, 2H), 1.36-1.52 (m, 4H), 2.01-2.05 (t, 2H), 2.56-2.60 (t, 2H), 2.60-3.05 (q, 2H), 3.23 (q, 2H), 6.09 (t, 2H), 6.68-6.79 (dd, 2H), 6.87 (t, 1H), 7.19 (t, 2H), 7.35-7.37 (d, 2H), 7.86 (t, 2H), 8.37 (s, 1H).

### ii. N-[6-[2-(4-Hydroxy-2-oxo-3H-1,3-benzothiazol-7-yl)-ethylamino]hexyl]-N'-phenyl urea hydrochloride

mp 209-210°;
Mass Spectrum: FAB 429 ((M+H)⁺) ;
nmr δ (d₆DMSO) 1.25-1.65 (m, 8H), 2.75-3.15 (m, 8H), 6.18-6.21 (t, 1H), 6.73-6.76 (d, 1H), 6.85-6.89 (t, 2H), 7.18-7.22 (t, 2H), 7.36-7.38 (d, 2H), 8.45 (brs, 3H), 10.13 (s, 1H), 11.75 (s, 1H);
Analysis: Found C,52.55; H,5.35; N,10.24; S,5.15%
C₂₁H₂₉N₄O₃S.HCl.0.64 CF₃CO₂H requires: C,51.99; H,5.55; N,10.41; S,5.96%.

### n) 4-Hydroxy-7-[2-[6-[2-(4-nitrophenyl)ethoxy]hexyl]amino-ethyl]-1,3-benzothiazol-2(3H)-one hydrochloride

Prepared by the method of Example 1b) using the amide of Example 11a).
mp 160-163°;
Mass spectrum: FAB 460 ((M+H)⁺);
nmr δ (d₆DMSO) 1.27 (m, 4H), 1.45 (m, 2H), 1.58 (m, 2H), 2.86 (m, 4H), 2.95 (t, 2H), 3.05 (brm, 2H), 3.38-3.62 (t, 2x2H), 6.76-6.86 (dd, 2H), 7.53-8.15 (dd, 4H), 8.88 (2H), 10.14 (s, 1H), 11.77 (s, 1H);
Analysis: Found C,55.35; H,6.08; N,8.48; S,6.62; Cl, 7.8%
C₂₃H₂₉N₃O₅S.HCl requires: C,55.69; H,6.09; N,8.47; S,6.42; C1,7.15%.

### o) 4-Hydroxy-7-[2-[6-[2-(4-hydroxyphenyl)ethoxy]-2,2-dimethylhexyl]aminoethyl]-1,3-benzothiazol-2-(3H)-one hydrochloride

### i. 6-[2-(4-Hydroxyphenyl)ethoxy]-N-[2-(4-hydroxy-2-oxo-3H-1,3-benzothiazol-7-yl)ethyl]-2,2-dimethylhexanamide

Mass Spectrum: FAB 473 ((M+H)⁺) ;
nmr δ (d₄MeOH) 0.98 (s)+1.00-1.05 (m) (8H), 1.29-1.37 (m, 4H), 2.60-2.65 (m, 4H), 3.25-3.47 (m, 6H+H₂O), 6.55-6.60 (m, 3H), 6.73-6.75 (d, 1H), 6.89-6.92 (dd, 2H).

### ii. 4-Hydroxy-7-[2-[6-[2-(4-hydroxyphenyl)ethoxy]-2,2-dimethylhexyl]aminoethyl]-1,3-benzothiazol-2(3H)-one hydrochloride

mp 204-205°;
Mass Spectrum: FAB 459 ((M+H)⁺);
nmr δ (d₆DMSO) 0.95 (s, 6H), 1.25 (brs, 4H), 1.45-1.48 (brt, 2H), 2.65-2.69 (t, 2H), 2.81 (brs, 2H), 2.90-2.95 (brm, 2H), 3.07 (brs, 2H), 3.37 (m, 2H+H₂O), 3.47-3.51 (t, 2H), 6.64-6.67 (d, 2H), 6.75-6.77 (d, 1H), 6.85-6.87 (d, 1H), 6.99-7.01 (d, 2H), 8.35 (brs, 2H), 9.2 (s, 1H), 10.1 (s, 1H), 11.8 (s, 1H);
Analysis: Found C,58.07; H,7.29; N,5.42; S,6.19%.
C₂₅H₃₄N₂O₄S.HCl (1.22M H₂O) requires: C,58.07; H,7.41; N,5.46; S,5.84%.

### p) 7-Hydroxy-4-[2-[6-[2-(1-naphthyl)ethoxy]hexyl]amino-ethyl]-1,3-benzothiazol-2(3H)-one hydrochloride

### i. N-[2-(4-Hydroxy-2-oxo-3H-1,3-benzothiazol-7-yl)ethyl]-6-[2-(1-naphthyl)ethoxy]hexanamide

Mass Spectrum: FAB 479 ((M+H)⁺).

### ii. 7-Hydroxy-4-[2-[6-[2-(1-naphthyl)ethoxy]hexyl]aminoethyl]-1,3-benzothiazol-2(3H)-one hydrochloride

mp 194-196°;
Mass Spectrum: FAB 465 ((M+H)⁺);
nmr δ (d₆DMSO) 1.27 (brs, 4H), 1.47 (brs, 2H), 1.57 (brs, 2H), 2.86 (brd, 4H), 3.04 (brs, 2H), 3.28 (t, 2H), 3.40 (t, 2H), 3.68 (t, 2H), 6.76 (d, 1H), 6.86 (d, 1H), 7.43 (m, 2H), 7.52 (m, 2H), 7.78 (d, 1H), 7.91 (d, 1H), 8.68 (d, 1H), 9.8 (brs, 2H), 10.13 (s, 1H), 11.77 (s, 1H) ;
Analysis: Found C,64.47; H,6.69; N,5.71; S,6.19%
C₂₇H₃₂N₂O₃S.HCl requires: C,64.71; H,6.64; N,5.59; S,6.40%.

### q) 7-[2-[6-(2-Cyclohexylethoxy)hexyl]aminoethyl]-4-hydroxy -1,3-benzothiazol-2(3H)-one hydrochloride

### i. 6-(2-Cyclohexylethoxy)-N-r2-(4-hydroxy-2-oxo-3H-1,3-benzothiazol-7-yl)ethyl]hexanamide

Mass Spectrum: FAB 435 ((M+H)⁺);
nmr δ (CDCl₃+d₆DMSO) 0.85 (m, 2H), 1.0-1.7 (m, 17H), 2.1 (t, 2H), 2.6 (t, 2H), 3.3 (m, 6H), 6.7 (d, 1H), 6.5 (d, 1H), 6.9 (t, 1H), 10.7 (brs, 1H);

### ii. 7-[2-[6-(2-Cyclohexylethoxy)hexyl]aminoethyl]-4-hydroxy -1,3-benzothiazol-2(3H)-one hydrochloride

mp 224-227°;
Mass Spectrum: FAB 421 ((M+H)^{+);}
nmr δ (d₆DMSO) 0.88 (m, 2H), 1.0-1.25 (m, 3H), 1.25-1.40 (m, 7H), 1.40-1.55 (m, 2H), 1.55-1.7 (m, 7H), 2.83 (m, 2H), 2.90 (m, 2H), 3.05 (m, 2H), 3.0-3.4 (m, 4H+H₂O), 6.75 (d, 1H), 6.86 (d, 1H), 8.7 (brs, 2H), 10.11 (s, 1H), 11.76 (brs, 1H);
Analysis: Found C,59.88; H,8.08; N,6.19; S,6.18; C1,8.69%
C₂₃H₃₆N₂O₃S.HCl requires: C,59.88; H,8.11; N,6.07; S,6.95; Cl,8.57%.

### r) 7-[2-[6-[2-(4-Bromophenyl)ethoxy]hexyl]aminoethyl]-4-hydroxy-1,3-benzothiazol-2(3H)-one hydrochloride

### i. 6-[2-(4-Bromophenyl)ethoxy]-N-[2-(4-hydroxy-2-oxo-3H-1,3-benzothiazol-7-yl)ethyl]hexanamide

mp 143-145°;
Mass Spectrum: FAB 507/9 ((M+H)⁺);
nmr δ (d₆DMSO) 1.15-1.24 (m, 2H), 1.42-1.49 (m, 4H), 2.01 (t, 2H), 2.59 (t, 2H), 2.76 (t, 2H), 3.18-3.30 (m, 2H), 3.33 (t, 2H), 3.53 (t, 2H), 6.70 (d, 1H), 6.79 (d, 1H), 7.19 (d, 2H), 7.45 (d, 2H), 7.85 (t, 1H), 9.5-11.5 (v.brs, 2H);
Analysis: Found C,54.39; H,5.30; N,5.63; S,5.14; Br,16.96%
C₂₃H₂₇N₂O₄SBr requires: C,54.44; H,5.36; N,5.52; S,6.32; Br,15.74%.

### ii. 7-[2-[6-[2-(4-Bromophenyl)ethoxy]hexyl]aminoethyl]-4-hydroxy-1,3-benzothiazol-2(3H)-one hydrochloride

mp 199-200°;
Mass Spectrum: FAB 577/9 ((M+Rb)⁺) ;
nmr δ (d₆DMSO) 1.22-1.37 (m, 4H), 1.42-1.51 (m, 2H), 1.57-1.64 (m, 2H), 2.77 (t, 2H), 2.85-2.96 (m, 4H), 3.06 (t, 2H), 3.36 (t, 2H+H₂O), 3.55 (t, 2H), 6.78 (d, 1H), 6.87 (d, 1H), 7.20 (d, 2H), 7.46 (d, 2H), 9.1 (v.brs, 1H), 10.2 (v.brs, 1H);
Analysis: Found C,51.98; H,5.68; N,5.39; S,5.94; Cl,7.02%
C₂₃H₂₉N₂O₃SBr.HCl requires: C,52.13; H,5.71; N,5.29; S,6.05; Cl,6.69%.

### s) 4-Hydroxy-7-[2-[6-[2-(2-thienyl)ethoxy]hexyl]amino-ethyl]-1,3-benzothiazol-2(3H)-one hydrochloride

### i. N-[2-(4-Hydroxy-2-oxo-3H-1,3-benzothiazol-7-yl)ethyl]-6-[2-(2-thienyl)ethoxy]hexanamide

mp 130-132°;
Mass Spectrum: FAB 435 ((M+H)⁺);
nmr δ (d₆DMSO) 1.16-1.29 (m, 2H), 1.40-1.57 (m, 4H), 2.01 (t, 2H), 2.59 (t, 2H), 3.00 (2H, t), 3.20-3.37 (m, 2H), 3.35-3.45 (m, 2H+H₂O), 3.56 (t, 2H), 6.70 (d, 1H), 6.79 (d, 1H), 6.89 (brs, 1H), 6.92-6.94 (m, 1H), 7.70 (d, 1H), 7.83-7.91 (m, 1H), 9.96 (brs, 1H), 11.58 (brs, 1H) ;
Analysis: Found C,57.76; H,6.06; N,6.64; S,14.48%
C₂₁H₂₆N₂O₄S₂ requires: C,58.04; H,6.03; N,6.45; S,14.48%.

### ii. 4-Hydroxy-7-[2-[6-[2-(2-thienyl)ethoxy]hexyl]amino-ethyl]-1,3-benzothiazol-2(3H)-one hydrochloride

mp 216-218°;
Mass Spectrum: FAB 421 ((M+H)⁺);
nmr δ (d₆DMSO) 1.31 (brs, 4H), 1.47-1.53 (m, 2H), 1.54-1.66 (m, 2H), 2.84-2.93 (m, 4H), 2.87 (t, 2H), 2.89 (brs, 2H), 3.40 (t, 2H), 3.57 (t, 2H), 6.77 (d, 1H), 6.87 (d, 1H), 6.89 (brs, 1H), 6.92-6.95 (m, 1H), 7.31 (dd, 1H), 9.01 (brs, 2H), 10.16 (s, 1H), 11.78 (s, 1H);
Analysis: Found C,55.40; H,6.47; N,6.28; S,13.86; Cl,7.38%
C₂₁H₂₈N₂O₃S₂.HCl requires: C,55.11; H,6.39; N,6.13; S,14.02; Cl,7.76%.

### t) (R,S)-4-Hydroxy-7-[2-[2-methyl-6-(2-phenylethoxy)hexyl] -aminoethyl]-1,3-benzothiazol-2(3H)-one hydrochloride

### i. N-[2-(4-Hydroxy-2-oxo-3H-1,3-benzothiazol-7-yl)ethyl]-2-methyl-6-(2-phenylethoxy)hexanamide

Mass Spectrum: 443 ((M+H)⁺).

### ii. (R,S)-4-Hydroxy-7-[2-[2-methyl-6-(2-phenylethoxy)hexyl] -aminoethyl]-1,3-benzothiazol-2(3H)-one hydrochloride

mp 211-212°;
Mass Spectrum: FAB 429 ((M+H)⁺) ;
nmr δ (d₆DMSO) 0.94 (d, 3H), 1.11-1.51 (m, 6H), 1.80-1.85 (m, 1H), 2.70-2.93 (m, 6H), 3.01-3.11 (m, 2H), 3.38 (t, 2H), 3.56 (t, 2H), 6.78 (d, 1H), 6.86 (d, 1H), 7.16-7.29 (m, 5H), 8.82 (brs, 2H), 10.15 (s, 1H), 11.78 (s, 1H) ;
Analysis: Found C,61.31; H,7.31; N,6.11; S,6.78%
C₂₄H₃₂N₂O₃S.HCl requires: C,61.98; H,7.15; N,6.02; S,6.98%.

### u) 1,3-Dihydro-4-hydroxy-7-[2-[6-(2-phenylethoxy)hexyl]-aminoethyl]-2H-benzimidazol-2-one hydrochloride

### i. N-[2-(1,3-Dihydro-4-hydroxy-2-oxo-2H-1,3-benzimidazol-7-yl)ethyl]-6-(2-phenylethoxy)hexanamide

Prepared by the method of Example 1a) from 7-(2-aminoethyl)-4-hydroxy-benzimidazol-2(3H)-one hydrobromide, which was prepared from N-[2-(3-amino-4-methoxyphenyl)ethyl]trifluoroacetamide using a procedure outlined in J. Med. Chem., 1987, 30, 1166.
mp 205-207°;
Mass Spectrum: FAB 412 ((M+H)⁺);
nmr δ (d₆DMSO) 1.18-1.24 (m, 2H), 1.43-1.49 (m, 4H), 2.01 (t, 2H), 2.65 (t, 2H), 2.79 (t, 2H), 3.21 (q, 2H), 3.34 (m, 2H+H₂O), 3.55 (t, 2H), 6.36 (d, 1H), 6.53 (d, 1H), 7.16-7.29 (m, 5H), 7.30 (t, 1H), 9.30 (s, 1H), 10.32 (s, 1H), 10.53 (s, 1H);
Analysis: Found C,64.34; H,7.14; N,9.92%
C₂₃H₂₉N₃O₄.0.88 H₂O requires: C,64.65; H,7.25; N,9.83%.

### ii. 1,3-Dihydro-4-hydroxy-7-[2-[6-(2-phenylethoxy)hexyl]-aminoethyl]-2H-benzimidazol-2-one hydrochloride

mp 222-224° ;
Mass Spectrum: FAB 398 ((M+H)⁺);
nmr δ (d₆DMSO) 1.22-1.33 (m, 4H), 1.43-1.51 (m, 2H), 1.53-1.65 (m, 2H), 2.79 (t, 2H), 2.81-2.92 (m, 4H), 3.01-3.09 (m, 2H), 3.38 (m, 2H+H₂O), 3.56 (t, 2H), 6.41 (d, 1H), 6.62 (d, 1H), 7.16-7.30 (m, 5H), 8.73 (brs, 2H), 9.54 (s, 1H), 10.48 (s, 1H), 10.65 (s, 1H);
Analysis: found C,63.30; H,7.52; N,6.67; Cl,8.11%
C₂₃H₃₁N₃O₃.HCl requires: C,63.65; H,7.43; N,6.68; Cl,8.17%.

### Example 3

### 4-Hydroxy-7-[2-[6-[2-(4-hydroxyphenyl)ethoxy]hexyl]-aminoethyl]-1,3-benzothiazol-2(3H)-one trifluoroacetate

### a) N-[2-(4-Hydroxy-2-oxo-3H-1,3-benzothiazol-7-yl)ethyl]-6-[2-(4-phenylmethoxy)phenylethoxy]hexanamide

The sub-titled amide was prepared according to the method of Example 1a).
mp 148-150°;
Mass Spectrum: FAB 535 (M++H)⁺);
nmr δ (d₆DMSO) 1.25-1.67 (m, 2H), 1.41-1.50 (m, 4H), 2.01 (t, 2H), 2.59 (t, 2H), 2.72 (t, 2H), 3.24 (q, 2H), 3.33 (t, 2H), 3.49 (t, 2H), 5.05 (s, 2H), 6.69-6.80 (2xd, 2H), 6.89-7.14 (dd, 4H), 7.30-7.44 (m, 5H), 9.92 (s, 1H), 11.63 (s, 1H).

### b) 4-Hydroxy-7-[2-[6-[2-(4-phenylmethoxy)phenylethoxy]-hexylamino]ethyl]-1,3-benzothiazol-2(3H)-one hydrochloride

The sub-titled compound was prepared according to the method of Example 1b), from the intermediate amide of step a).
mp 228-230°;
nmr δ (d₆DMSO) 1.29 (brs, 4H), 1.41-1.49 (m, 2H), 1.61 (brs, 2H), 2.70-2.74 (t, 2H), 2.85-2.90 (m, 4H), 3.05 (brs, 2H), 3.34-3.38 (t, 2H), 3.48-3.52 (t, 2H), 5.06 (s, 2H), 6.77-6.88 (2xd, 2H), 6.90-6.93+7.13-7.15 (dd, 4H), 7.30-7.43 (m, 5H), 8.9-9.0 (brs, 2H), 10.18 (brs, 1H), 11.80 (s, 1H);
Analysis: Found C,64.84; H,6.80; N.5.17; S,5.55%
C₃₀H₃₆N₂O₄S.HCl requires: C,64.69; H,6.64; N,5.03; S,5.75%.

### c) 4-Hydroxy-7-[2-[6-[2-(4-hydroxyphenyl)ethoxy]hexyl]-aminoethyl]-1,3-benzothiazol-2(3H)-one trifluoroacetate

A solution of the benzyl ether from step b) (0.79 g) in methanol (50 ml) was treated with conc. hydrochloric acid to pH 1 and hydrogenated over 10% palladium on carbon (0.50 g) for 8 days, the catalyst being replaced with a fresh portion after 7 days. The mixture was diluted with methanol (100 ml) boiled for 10 min and hot filtered. The filtrate was evaporated to give a yellow semi-solid. HPLC using methanol:0.1% TFA in water (50:50) as eluant, gave the title salt as a white powder.
mp 192-197°;
nmr δ (d₆DMSO) 1.29 (brs, 4H), 1.48-1.56 (brm, 4H), 2.65-2.67 (t, 2H), 2.80-2.82 (t, 2H), 2.90+3.08 (brs, 4H), 3.36-3.38 (t, 2H+H₂O), 3.46-3.50 (t, 2H), 6.66+7.00 (dd, 4H), 6.75+6.86 (2xd, 2H);
Analysis: Found C,55.01; H,5.85; N,5,12; S,5.29%
C₂₃H₃₀N₂O₄S.CF₃CO₂H requires: C,55.24; H,5.56; N,5.15; S,5.90%.

### Example 4

### 2,3-Dihydro-7-hydroxy-4-[2-[6-(phenylethoxy)hexyl]- aminoethyl]-indol-2-one hydrochloride

### a) N-[2-(3-Nitro-4-phenylmethoxy)phenyl]ethyl-N-phenyl-methyl-6-(2-phenylethoxy)hexanamide

A solution of N,O-bis-(phenylmethyl)-2-(4-hydroxy-3-nitrophenyl)ethylamine (7.5 g), 6-[2-phenylethyoxy]-hexanoic acid (5.38 g) and carbonyldiimidazole (3.69 g) in dichloromethane (120 ml) was stirred at room temperature for 20 hours. Aqueous 5% hydrochloric acid (50 ml) was added and the mixture filtered, the organic layer was separated, washed with brine, (NaHCO₃), and again with brine. After drying (MgSO₄) the solvent was removed under reduced pressure and the residue purified by chromatography (SiO₂) using ether/60-80* petroleum ether as eluant, to give the sub-titled compound as a yellow oil.
Mass Spectrum: FAB 581 ((M+H)⁺).

### b) N-[2-(3-Amino-4-phenylmethoxy)phenyl]ethyl-N-phenyl-methyl-6-(2-phenylethoxy)hexanamide

The amide from step a) (7.0 g) was added to a suspension of iron powder (7.0 g) and ammonium chloride (7.0 g) in aqueous ethanol (90 ml ethanol:70 ml water). The suspension was stirred and heated to reflux for 2 hours. The hot reaction mixture was filtered (Hyflo Supergel) and the filtrate evaporated under reduced pressure. The residue was partitioned between dichloromethane and water, the organic layer was separated, dried (MgSO₄) and evaporated under reduced pressure to give the sub-titled compound as a yellow gum which was used without further purification.
Mass Spectrum: FAB 551 ((M+H)⁺);
nmr δ (CDCl₃) 1.35 (m, 2H), 1.57-1.65 (m, 4H), 2.28 (m, 2H), 2.65-2.73 (m, 2H), 2.88 (m, 2H), 3.40 (m, 2H), 3.51 (t, 2H), 3.61 (m, 2H), 3.91 (brs, 2H), 4.35 (s, 1H), 4.59 (s, 1H), 5.04 (s, 2H), 6.42-6.57 (m, 2H), 6.75 (d, 1H), 7.10 (d, 1H), 7.21-7.41 (m, 14H).

### c) 3-Amino-N-[6-(2-phenylethoxy)hexyl)hexyl-4-phenylmethoxy-N-phenylmethyl 2-benzeneethanamide

Borane (1M in THF, 20 ml) was added to a stirred solution of the compound from step b) (6.27 g) in tetrahydrofuran. The solution was heated under reflux for 18 hours, cooled and methanol (5 ml) added. The solvent was removed under reduced pressure and the residue dissolved in methanol. Ethanolic HCl was added and the solution heated under reflux for 40 min, the solvent removed under reduced pressure, and the compound converted to the free base which was used in the next step without further purification.
Mass Spectrum: FAB 537 ((M+H)⁺).

### d) 1,3-Dihydro-3-methylthio-4-[2-[N-[6-(2-phenylethoxy)-hexyl]-N-(phenylmethyl)amino]ethyl]-7-phenylmethoxy)-2H-indol-2-one

Sulfuryl chloride (0.81 ml) in dichloromethane was added dropwise to a solution of ethyl methylthioacetate in dichloromethane (25 ml) at -74°. The solution was stirred for 45 min and a solution of the compound from step c) (4.35 g) and Proton Sponge™ (1.74 g) in dry dichloromethane (50 ml) added dropwise. The mixture was stirred at -74° for 4 hours then triethylamine (1.17 ml) added. The mixture was allowed to warm to room temperature and stirred overnight. Aqueous 5% hydrochloric acid (35 ml) was added and stirring continued for 1 hour. The organic layer was separated, dried (MgSO₄) and the solvent removed under reduced pressure. Chromatography (SiO₂) using methanol/dichloromethane as eluant gave the sub-titled compound as a green oil.
Mass Spectrum: FAB 623 ((M+H)⁺).

### e) 1,3-Dihydro-4-[2-[N-[6-(2-phenylethoxy)hexyl]-N-(phenyl -methyl)amino]ethyl]-7-phenylmethoxy-2Hindol-2-one

Raney nickel (1.5 g) was added to a solution of the compound from step d) (2.1 g) in dry ethanol (60 ml). The mixture was heated to reflux for 1 hour, the catalyst was removed by filtration and the filtrate evaporated under reduced pressure. Trituration with ether gave an off-white solid which was purified by reverse phase HPLC (SiO₂) using methanol:chloroform as eluant. Recrystallisation from isopropyl alcohol gave the sub-titled compound as a buff coloured solid.
mp 183-185°;
Mass Spectrum: FAB 577 ((M+H)⁺);
nmr δ (CDCl₃) 1.34 (m, 4H), 1.55 (m, 2H), 1.90 (m, 2H), 2.87 (t, 2H), 2.8-3.2 (m, 6H), 3.3-3.5 (q, 4H), 3.61 (t, 2H), 4.22 (d, 2H), 5.07 (s, 2H), 6.77 (q, 1H), 7.20 (q, 3H), 7.29 (m, 3H), 7.38 (m, 5H), 7.47 (t, 3H), 7.61 (s, 1H), 7.66 (q, 2H).
Mass Spectrum: FAB 577 ((M+H)⁺);

### f) 1,3-Dihydro-7-hydroxy-4-[2-[6-(phenylethoxy)hexyl]-aminoethyl]-2H-indol-2-one hydrochloride

The compound from step e) (0.18 g) was dissolved in dry ethanol (50 ml) containing 2 drops of conc. hydrochloric acid (sg. 1.18). The mixture was hydrogenated at atmospheric pressure using 5% Pd/C (0.020 g) as catalyst. After 4 hours the catalyst was removed by filtration and the solvent evaporated under reduced pressure, recrystallisation from ethanol gave the title compound as a white solid.
mp 277-278° (decomposes);
Mass Spectrum: Thermal Spray Plasma 397 ((M+H)⁺);
nmr δ (d₆DMSO) 1.28 (m, 4H), 1.49 (t, 2H), 1.59 (t, 2H), 2.72-2.82 (m, 4H), 2.88 (t, 2H), 3.02 (t, 2H), 3.36 (2H+H₂O), 3.48 (s, 2H), 3.56 (t, 2H), 6.66 (q, 2H), 7.21-7.28 (m, 5H), 8.75 (brs, 2H), 9.45 (s, 1H), 10.22 (s, 1H) ;
Analysis: Found C,65.96; H,7.67; N,6.58%
C₂₃H₃₂N₂O₃.HCl requires: C,66.51; H,7.68; N,6.47%.

### Example 5

### 4-Hydroxy-7-[2-[3-[2-(2-phenylethyl)aminoethyl]-sulphonylpropyl]aminoethyl]-1,3-benzothiazol-2(3H)-one dihydrochloride

### a) 3-[2-Oxo-2-(2-phenylethyl)amino]ethylthiopropanoic acid

A solution of 3-mercaptopropanoic acid (1.63 ml) in dimethylformamide (5 ml) was added gradually to a stirred suspension of sodium hydride (2 eq.) in dimethylformamide at room temperature. The suspension was stirred for 2 hours then cooled to 0° before gradually adding a solution of chloro-N-(2-phenylethyl)acetamide (3.7 g) in dimethylformamide and allowing the mixture to warm to room temperature. After stirring overnight the solvent was removed under reduced pressure and the residue taken up in ethyl acetate. The organic layer was washed with dilute aqueous hydrochloric acid and brine. Drying and evaporation of the solvent under reduced pressure gave a yellow oil which was purified by column chromatography (SiO₂).
Mass Spectrum: 339 (mono-TMS derivative);
nmr δ (CDCl₃) 2.57 (t, 2H), 2.69 (t, 2H), 2.85 (m, 2H), 3.22 (s, 2H), 3.53-3.58 (q, 2H), 7.14-7.34 (m, 6H).

### b) N-[2-(2,4-Dimethoxy-1,3-benzothiazol-7-yl)ethyl]-3-[2-oxo-2-(2-phenylethylamino)ethylthio]propanamide

Carbonyldiimidazole (3.04 g) was added to a solution of the compound from step a) (4.55 g) in dichloromethane (30 ml) and the mixture stirred at room temperature for 90 min. 2-(2,4-dimethoxy-1,3-benzothiazol-7-yl)-ethylamine (4.06 g) in dichloromethane was added and the mixture stirred overnight. Water was then added and the aqueous phase extracted with dichloromethane. The organic extracts were washed (dilute HCl, NaHCO₃ and brine), dried (MgSO₄) and the solvent removed under reduced pressure to yield the sub-titled compound as an orange oil which was used without further purification.
Mass Spectrum: FAB 488 ((M+H)⁺).

### c) N-[2-(4-Methoxy-2-oxo-3H-1,3-benzothiazol-7-yl)-ethyl]-3-[2-oxo-2-(2-phenylethylamino]ethylthio]propanamide

Conc. hydrochloric acid (sg. 1.18, 1.5 ml) was added to the compound from step b) (3.3 g) in methanol (30 ml) and the mixture stirred at room temperature overnight. The solvent was evaporated under reduced pressure and the residue partitioned between chloroform and water. The organic layer was separated and the aqueous layer extracted with dichloromethane. The combined organic extracts were dried (MgSO₄) and the solvents removed under reduced pressure to give the sub-titled compound as an orange oil.
Mass Spectrum: FAB 474 ((M+H)⁺);
nmr δ (CDCl₃) 2.38-3.77 (m, 14H), 3.82-3.92 (m, 3H), 6.27 (brs, 1H), 6.74 (d, 1H), 6.93 (d, 1H), 7.1 (brs, 1H), 7.15-7.33 (m, 5H), 9.05 (s, 1H).

### d) N-[2-(4-Methoxy-2-oxo-3H-1,3-benzothiazol-7-yl)ethyl]--3-[2-oxo-2-(2-phenylethylamino)ethylsulphonyl]propanamide

Potassium peroxymonosulphate (5.57 g) was gradually added to a solution of the compound from step c) (2.81 g) in methanol:water (6:4, 72 ml) cooled to 0°. The mixture was stirred for 4 hours, water added and the whole extracted with chloroform. A yellow solid precipitated from the aqueous layer and was removed by filtration, and washed (water and pentane) to give the sub-titled compound.
Mass Spectrum: FAB 506 ((M+H)⁺);
nmr δ (d₆DMSO) 2.60-2.78 (2xt, 4H), 3.24-3.39 (m, 4H), 3.45-3.55 (m, 4H), 3.84 (s, 3H), 4.08 (s, 2H), 6.92 (d, 1H), 6.96 (d, 1H), 7.18-7.32 (m, 5H), 8.18 (brt, 1H), 8.44 (brt, 1H), 11.86 (s, 1H).

### e) 4-Methoxy-7-[2-N-[3-[2-N'-(2-phenylethyl)aminoethyl]-sulphonylpropyl]aminoethyl]-1,3-benzothiazol-2(3H)-one dihydrochloride

Prepared by the method of Example 1b).
Mass Spectrum: FAB 478 ((M+H)⁺).

### f) 4-Hydroxy-7-[2-[3-[2-(2-phenylethyl)aminoethyl]-sulphonylpropyl]aminoethyl]-1,3-benzothiazol-2(3H)-one dihydrochloride

The compound from step e) (0.33 g) was dissolved in 48% aqueous hydrobromic acid (10 ml) to which hypophosphorous acid (2 drops) had been added and the mixture heated to reflux under argon for 3 hours. The hydrobromic acid was removed under reduced pressure and the residue purified by reverse phase HPLC using methanol/0.1% aqueous trifluoroacetic acid as eluant, to give the title compound.
mp 241-244°;
nmr δ (d₆DMSO) 2.03-2.15 (m, 2H), 2.86 (t, 2H), 2.96 (t, 2H), 3.02-3.18 (brm, 4H), 3.22 (t, 2H), 3.40-3.45 (m, 4H), 3.62 (t, 2H), 6.76 (d, 1H), 6.86 (d, 1H), 7.23-7.39 (m, 5H), 8.93 (brs, 2H), 9.16 (brs, 2H), 10.11 (s, 1H), 11.76 (s, 1H);
Analysis: Found C,43.99; H,5.27; N,6.74; S,10.30%
C₂₂H₂₉N₃O₄S₂.1.8HCl requires: C,43.88; H,5.49; N,6.98; S,10.64%.

### Example 6

### (R,S)-4-Hydroxy-7-[2-[7-(2-phenylethoxy)-hept-2-yl]-aminoethyl]-1,3-benzothiazol-2(3H)-one hydrochloride

Sodium cyanoborohydride (0.056 g), 6-(2-phenylethoxy-oxy)-heptan-2-one (1.450 g) and 6% aqueous acetic acid (20 drops) were added to a solution of 7-(2-aminoethyl)-4-hydroxy-1,3-benzothiazol-2(3H)-one hydrochloride (0.300 g) in methanol (30 ml). The mixture was stirred for 4 days at room temperature until HPLC, using methanol/0.1% aqueous trifluoroacetate as eluant, revealed that all the starting material had been consumed. Aqueous ammonium hydroxide (2 drops) was added and the solvent removed under reduced pressure. Purification by chromatography (SiO₂) using ethanol/dichloromethane as eluant followed by reverse phase HPLC, using methanol/0.1% aqueous trifluoroacetate as eluant, and formation of the hydrochloride salt gave the title compound as a white solid.
mp 166-168° ;
Mass Spectrum: FAB 429 ((M+H)⁺);
nmr δ (d₆DMSO) 1.20-1.40 (m, 5H), 1.20 (d, 3H), 1.40-1.60 (m, 2H), 1.70 (brm, 1H), 2.79 (t, 2H), 2.88 (m, 2H), 3.00-3.20 (brm, 3H), 3.37 (t, 2H), 3.55 (t, 2H), 6.76 (d, 1H), 6.89 (d, 1H), 7.15-7.30 (m, 5H), 8.81 (brs, 1H), 8.90 (brs, 1H), 10.14 (s, 1H), 11.77 (s, 1H);
Analysis: Found C,61.45; H,7.67; N,6.17; S,7.06%
C₂₄H₃₂N₂O₃S.HCl requires: C,61.99; H,7.15; N,6.02; S,6.89%.

### Example 7

### 6-[2-[(4-Hydroxy-2-oxo-3H-1,3-benzothiazol-7-yl)ethyl]-amino]-N-(2-phenylethyl)hexanamide hydrochloride

### a) 6-[N'-(2-Chloro-4-methoxy-1,3-benzothiazol-7-yl)ethyl]-N'-[(2,2,2-trifluoroacetyl)amino]-N-(2-phenylethyl)-hexanamide

Sodium hydride (80% in oil, 0.244 g) was added to a stirred solution of N-[2-(2-chloro-4-hydroxy-1,3-benzothiazol-7-yl)ethyl]trifluoroacetamide (2.5 g) at room temperature. After stirring for 10 min the reaction was heated to 65° for 90 min. The mixture was cooled and a solution of 6-bromo-N-(2-phenylethyl)hexanamide (2.42 g) in dimethylformamide added, the mixture was stirred for 16 hours then heated to 70° for 3.5 hours. The solvent was evaporated under reduced pressure and the residue partitioned between dilute aqueous HCl and ethyl acetate. The organic layer was washed with brine, dried (MgSO₄) and the solvent removed under reduced pressure. Chromatography (SiO₂) using ethyl acetate/dichloromethane as eluant gave the sub-titled compound as a pale yellow oil.
Mass Spectrum: FAB 556 ((M+H)⁺) ;
nmr δ (CDCl₃) 1.20-1.70 (m, 6H), 2.05-2.18 (m, 2H), 2.81 (t, 2H), 2.87-3.07 (m, 2H), 3.20+3.39 (2xt, 2H), 3.49-3.56 (q, 2H), 3.59-3.66 (m, 2H), 4.01+4.02 (2xs, 3H), 5.45 (brs, 1H), 6.89 (t, 1H), 7.15-7.34 (m, 6H).

### b) 6-[2-[(2,4-Dimethoxybenzothiazol-7-yl)ethyl]amino]-N-(2-phenylethyl)hexanamide

The compound from step a) (1.73 g) in methanol was added to a solution of sodium methoxide in methanol (Na metal 0.17 g in methanol 40 ml). The mixture was heated under reflux for 4.5 hours, water (6 ml) was added and the mixture heated under reflux for a further 18 hours. The solvent was removed under reduced pressure, water added and the residue acidified with glacial acetic acid then made basic with ammonium hydroxide solution. The solution was extracted with chloroform, the organic layers dried (Na₂SO₄) and the solvent removed under reduced pressure. Chromatography (SiO₂) using methanol/dichloromethane as eluant gave the sub-titled compound as a pale yellow solid.
mp 112-113°;
Mass Spectrum: FAB 456 ((M+H)⁺);
nmr δ (CDCl₃) 1.23-1.33 (m, 2H), 1.40-1.65 (m, 2H), 2.11 (t, 2H), 2.61 (t, 2H), 2.84 (t, 2H), 2.85-3.00 (m, 6H), 3.50-3.57 (q, 2H), 3.99 (s, 3H), 4.24 (s, 3H), 5.45 (brs, 1H), 6.82 (d, 1H), 7.02 (d, 1H), 7.17-7.3 (m, 5H);
Analysis: Found C,66.25; H,7.38; N,9.39; S,6.82%
C₂₅H₃₃N₃O₃S requires: C,65.90; H,7.30; N,9.22; S,7.04%.

### c) 6-[2-(4-Hydroxy-2-oxo-3H-1,3-benzothiazol-7-yl)ethyl-aminol-N-(2-phenylethyl)hexanamide hydrochloride

Boron tribromide (1M in dichloromethane, 15 ml) was added dropwise to a solution of the compound from step b) (0.53 g) in dichloromethane (30 ml). The mixture was stirred at room temperature for 48 hours and a further portion of boron tribromide solution (7 ml) added. Stirring was continued for 24 hours, methanol added to destroy any remaining boron tribromide and the solvent removed under reduced pressure. A portion of toluene was added and then removed under reduced pressure. The residue was purified by reverse phase HPLC using methanol/0.1% aqueous trifluoroacetic acid as eluant. Conversion to the hydrochloride salt gave the title compound as a white solid.
mp 206-208
Mass Spectrum: FAB 428 ((M+H)⁺) ;
nmr δ (d₆DMSO) 1.20-1.30 (m, 2H), 1.42-1.53 (q, 2H), 1.50-1.65 (q, 2H), 2.05 (t, 2H), 2.70 (t, 2H), 2.80-2.95 (m, 4H), 3.00-3.12 (brm, 2H), 3.22-3.31 (q, 2H), 6.77 (d, 1H), 6.87 (d, 1H), 7.15-7.30 (m, 5H), 7.92 (t, 1H), 8.87 (brs, 2H), 10.15 (brs, 1H), 11.77 (s, 1H);
Analysis: Found C,54.71; H,6.08; N,8.22; S,5.88; Cl,12.34%
C₂₃H₂₉N₃O₃S.2HCl requires: C,55.19; H,6.24; N,8.40; S,6.41; Cl,14.16%.

### Example 8

### 4-Hydroxy-7-[2-[6-(2-phenylethylthio)hexyl]aminoethyl]-1.3-benzothiazol-2(3H)-one hydrochloride

### a) 6-(2-Phenylthio)hexanoic acid 7-r2-r6-(2-phenylethyl- thio)hexanamido]ethyl]-1,3-benzothiazol-2(3H)-one ester

Triethylamine (1 ml) was added to a suspension of 7-(2-aminoethyl)-4-hydroxy-1,3-benzothiazol-2(3H)-one hydrobromide (2.07 g) in chloroform (30 ml). Triethylamine (2 ml) followed by ethyl chloroformate (1.33 ml) were added to a solution of 6-(2-phenylethylthio)hexanoic acid (3.35 g) in chloroform (30 ml) and the mixture stirred for 30 min. The two solutions were then mixed and stirred for 72 hours at room temperature. Water was added and the aqueous layer extracted with dichloromethane, the organic layers were washed with brine, dried (MgSO₄) and the solvent removed under reduced pressure. Chromatography (SiO₂) using methanol/dichloromethane as eluant gave the sub-titled compound as a white solid.
mp 178-178.5°;
Mass Spectrum: FAB 679 ((M+H)⁺) ;
nmr δ (CDCl₃) 1.23-1.29 (m, 2H), 1.42-1.68 (m, 10H), 1.99-2.04 (t, 2H), 2.45-2.84 (m, 16H), 3.27-3.33 (m, 2H), 6.99 (s, 2H), 7.18-7.30 (m, 10H), 7.90-7.93 (t, 1H), 12.16 (s, 1H);
Analysis: Found C,65.65; H,7.02; N,4.17; S,13.71%
C₃₇H₄₆N₂O₄S₃ requires: C,65.48; H,6.78; N,4.12; S,14.15%.

### b) 4-Hydroxy-7-[2-[6-(2-phenylethylthio)hexyl]aminoethyl]-1,3-benzothiazol-2(3H)-one hydrochloride

Prepared by the method of Example 1b) using the compound from step a).
mp 226.5-227.5°;
nmr δ (d₆DMSO) 1.25-1.4 (m, 4H), 1.45-1.65 (m, 4H), 2.7-2.95 (m, 8H), 3.0-3.1 (m, 2H), 3.34 (brs, 2H), 6.75+6.88 (2xd, 2H), 7.17-7.30 (m, 5H), 8.84 (brs, 2H), 10.14 (s, 1H), 11.77 (s, 1H);
Analysis: Found C,59.46; H,6.85; N,6.08; S,13.35%
C₂₃H₃₀N₂O₂S₂.HCl requires: C,59.14; H,6.69; N,6.00; S,13.73%.

### Example 9

### N-[2-Hydroxy-5-[2-N'-[6-(2-phenylethoxy)hexyl]amino-ethyl]phenyl]formamide oxalate salt

### a) N-[2-(3-Nitro-4-phenylmethoxyphenyl)ethyl]trifluoro-acetamide

Benzyl bromide (7.86 g) was added to a stirred solution of N-[2-(3-nitro-4-phenol)ethyl]trifluoroacetamide (11.66 g) and anhydrous potassium carbonate (6.3 g) in dimethylformamide (100 ml), cooled in an ice bath. Stirring was continued for 5 hours and the mixture poured into ice/water. The solid produced was dissolved in ethyl acetate and the aqueous phase extraced with ethyl acetate. The organic extracts were washed with brine, dried and the solvent removed under reduced pressure. The residue was purified by column chromatography to give the sub-titled compound as a white crystalline solid.
mp 85-86°;
Mass Spectrum: RbI 453/5 ((M+Rb)⁺) ;
nmr δ (CDCl₃) 2.87 (t, 2H), 3.58 (m, 2H), 5.20 (s, 2H), 6.56 (br, 1H), 7.07 (d, 1H), 7.3-7.5 (m, 6H), 7.69 (d, 1H) ;
Analysis: Found C,55.95; H,4.28; N,7.63; F,15.98%
C₁₇H₁₅F₃N₂O₄ requires: C,55.43; H,4.10; N,7.65; F,15.47%.

### b) 3-Nitro-4-(phenylmethoxy)benzeneethanamine hydrochloride

The compound from step a) (9.8 g), and anhydrous potassium carbonate (4.03 g) in water (10 ml) and ethanol (100 ml) were heated to reflux for 2 hours. The ethanol was evaporated under reduced pressure, water was added and the mixture extracted with ethyl acetate. The organic extracts were dried and the solvent removed under reduced pressure and the residue converted to the hydrochloride salt.
mp 190-193°;
Mass Spectrum: FAB 272 ((M+H)⁺);
nmr δ (d₆DMSO) 2.92 (t, 2H), 3.04 (t, 2H), 5.30 (s, 2H), 7.3-7.5 (m, 6H), 7.57 (dd, 1H), 7.83 (d, 1H), 8.15 (brs, 3H);
Analysis: Found C,56.68; H,5.43; N,8.84; Cl,11.14%
C₁₅H₁₆N₂O₃.HCl requires: C,56.68; H,5.72; N,8.79; Cl,11.13%.

### c) N-2-[3-Nitro(4-phenylmethoxy)phenyl]ethyl-6-(2-phenylethoxy)hexanamide

Carbonyldiimidazole (0.33 g) was added to a solution of 6-(2-phenylethoxy)hexanoic acid (0.47 g) in dichloromethane, the mixture was stirred at room temperature for 1 hour. A solution of the compound from step b) (0.54 g) was then added and the mixture stirred for 1 hour. Water was added and the organic layer separated and washed with dilute HCl and brine, dried (MgSO₄) and the solvent removed under reduced pressure. The residue was purified by column chromatography.
Mass Spectrum: FAB 491 ((M+H)⁺);
nmr δ (CDCl₃) 1.33 (m, 2H), 1.59 (m, 4H), 2.1 (t, 2H), 2.82/2.86 (t, 2x2H), 3.41-3.61 (m, 3x2H), 5.21 (s, 2H), 7.04 (d, 1H), 7.15-7.6 (m, 11H), 7.66 (d, 1H).

### d) 3-Nitro-4-phenylmethoxy-N-[6-(2-phenylethoxy)hexyl]-benzeneethanamine hydrochloride

Prepared by the method of Example 1b) using the compound from step c).
mp 142-143°;
Mass Spectrum: FAB 477 ((M+H)⁺) ;
nmr δ(d₆DMSO) 1.45 (brm, 4H), 1.47 (brt, 2H), 1.60 (brt, 2H), 2.80 (t, 2H), 2.85 (brm, 2H), 3.00 (m, 2H), 3.12 (brm, 2H), 3.37 (t, 2H), 3.55 (t, 2H), 5.30 (s, 2H), 7.15-7.45 (m, 11H), 7.57 (dd, 1H), 7.84 (d, 1H), 8.97 (brs, 2H);
Analysis: Found C,67.52; H,7.44; N,5.71; C1,6.90%
C₂₉H₃₆N₂O₄.HCl requires: C,67.88; H,7.27; N,5.46; Cl,6.91%.

### e) 3-Nitro-N-[6-(2-phenylethoxy)hexyl]-4-(phenylmethoxy)-N-(phenylmethoxycarbonyl)benzeneethanamine

Triethylamine (0.165 g) was added to a stirred suspension of the compound from step d) (0.25 g) in dichloromethane (20 ml) cooled in ice. Benzylchloroformate (0.1 g) was added and the mixture allowed to warm to room temperature. The mixture was stirred until TLC indicated the reaction was complete then a further equivalent of benzylchloroformate was added and the mixture stirred for a 2 hours. Water was added, the organic layer was separated, dried and the solvent removed under reduced pressure to give the sub-titled compound as a yellow oil which was used without further purification.
Mass Spectrum: TSP with NH₄⁺ 628 ((M+NH₄)⁺);
nmr δ (CDCl₃) 1.28-1.52 (m, 6H), 2.8-2.89 (m, 6H), 3.17 (m, 2H), 3.41 (m, 4H), 3.60 (t, 2H), 4.59 (s, 2H), 5.07-5.20 (m, 2H), 7.20-7.60 (m, 18H).

### f) 3-Amino-4-(phenylmethoxy)-N-[6-(2-phenylethoxy)hexyl]-N-(phenylmethoxycarbonyl)benzeneethanamine

Prepared by the method of Example 4b), and the resulting oil purified by column chromatography (SiO₂) using diethyl ether:petrol (1:1) as eluant.
Mass Spectrum: FAB 581 ((M+H)⁺);
nmr δ (CDCl₃) 1.25 (m, 4H), 1.51 (m, 4H), 2.70 (m, 2H), 2.87 (t, 2H), 3.17 (m, 2H), 3.40 (m, 4H), 3.60 (t, 2H), 5.04 (m, 2H), 5.12 (m, 2H), 6.4-6.8 (m, 3H), 7.15-7.50 (m, 15H).

### g) N-5-[2-N'-[6-(2-Phenylethoxy)hexyl]aminoethyl]-N'-[(phenylmethoxycarbonyl)-2-(phenylmethoxy)phenyl]formamide

The compound from step f) (0.58 g) was heated under reflux for 21 hours in neat ethylformate. The reaction was evaporated to dryness and the residual oil purified by column chromatography using ethyl acetate:petrol (1:3) as eluant.
Mass Spectrum: FAB 609 ((M+H)⁺);
nmr δ (CDCl₃) 1.25 (m, 4H), 1.51 (m, 4H), 2.78 (m, 2H), 2.87 (t, 2H), 3.2-3.4 (m, 6H), 3.6 (t, 2H), 5.06 (s, 2H), 5.12 (d, 2H), 6.85 (m, 2H), 7.15-7.45 (m, 16H), 7.6-7.75 (brs, 1H), 8.26-8.38 (d, 1H).

### h) N-[2-Hydroxy-5-[2-N'-[6-(2-phenylethoxy)hexyl]amino-ethyl]phenyl]formamide oxalate salt

A mixture of the compound from step g) (0.3 g), ammonium formate (0.3 g) and 10% Pd/C (0.3 g) in methanol (30 ml) was heated at reflux for 30 min. The mixture was filtered (Hyflo Supergel) and the solvent evaporated under reduced pressure. The resulting solid was purified by column chromatography (SiO₂) using dichloromethane:methanol (4:1) as eluant. The free base of the title compound was then converted to the oxalate salt.
mp 207-209°;
Mass Spectrum: FAB 385 ((M+H)⁺);
nmr δ (d₆DMSO) 1.28 (m, 4H), 1.47 (m, 2H), 1.55 (m, 2H), 2.79 (m, 4H), 2.85 (m, 2H), 3.00 (m, 2H), 3.37-3.55 (m, 4H+H₂O), 6.82 (m, 2H), 7.23 (m, 6H), 7.99 (br, 1H), 8.27 (br, 1H), 8.6 (brs, 2H), 9.6 (brs, 1H);
Analysis: Found C,57.94; H,6.93; N,6.02%
C₂₃H₃₂N₂O₃.C₂H₂O₄(0.9M oxalate) requires: C,57.94; H,6.45; N,6.04%.

### Example 10

### 7-[2-[6-[2-(4-Aminophenyl)ethoxy]hexyl]aminoethyl]-4-hydroxy-1.3-benzothiazol-2(3H)-one dihydrochloride

### a) N-[2-(4-Hydroxy-2-oxo-3H-1,3-benzothiazol-7-yl)ethyl]-6-[2-(4-nitrophenyl)ethoxy]hexanamide

Prepared by the method of Example 1a), using 6-[2-(4-nitrophenyl)ethyl]hexanoic acid and 7-(2-aminoethyl)-4-hydroxy-1,3-benzothiazol-2(3H)-one hydrobromide.
Mass Spectrum: FAB 474 ((M+H)⁺);
nmr δ (d₆DMSO) 1.2 (m, 2H), 1.45 (m, 4H), 2.0 (t, 2H), 2.55 (t, 2H), 2.95 (t, 2H), 3.25 (m, 2H), 3.35 (t, 2H), 3.62 (t, 2H), 6.70 (d, 1H), 6.79 (d, 1H), 7.50-8.15 (m, 4H) ;
Analysis: Found C,58.18; H,5.99; N,9.01; S,6.57%
C₂₃H₂₇N₃O₆S requires: C,58.33; H,5.75; N,8.87; S,6.77%.

### b) 6-[2-(4-Aminophenyl)ethoxy]-N-[2-(4-hydroxy-2-oxo-3H-1,3-benzothiazol-7-yl)ethyl]hexanamide

The compound of step a) (0.5 g), hydrazine hydrate (5 ml) and 10% Pd/C (0.05 g) in ethanol (50 ml) were heated at reflux for 2 hours. The mixture was filtered (Hyflo Supergel), the solvent removed under reduced pressure and the residue purified by chromatography using dichloromethane: methanol (9:1) as eluant.
Mass Spectrum: FAB 444 ((M+H)⁺);
nmr δ (d₆DMSO) 1.2 (m, 2H), 1.45 (m, 4H), 2.0 (t, 2H), 2.6 (m, 4H), 3.25 (m, 2H), 3.3-3.45 (t, 2x2H), 4.82 (brs, 2H), 6.45-6.85 (dd, 4H), 6.7-6.8 (dd, 2H), 7.84 (t, 1H), 9.89 (brs, 1H), 11.6 (brs, 1H);
Analysis: Found C,62.45; H,6.70; N,9.64; S,6.76%
C₂₃H₂₉N₃O₄S requires: C,62.28; H,6.59; N,9.47; S,7.22%.

### c) 7-[2-[6-[2-(4-Aminophenyl)ethoxy]hexyl]aminoethyl]-4-hydroxy-1,3-benzothiazol-2(3H)-one dihydrochloride

Prepared by the method of Example 1b), using the compound from step b).
mp 154-157°;
Mass Spectrum: FAB 430 ((M+H)⁺) ;
nmr δ (d₆DMSO) 1.25 (m, 4H), 1.45 (m, 2H), 1.58 (m, 2H), 2.6-2.9 (m, 6H), 3.05 (m, 2H), 3.35 (t, 2H), 3.55 (t, 2H), 6.78-6.88 (dd, 2H), 7.3 (m, 4H), 9.05 (brs, 2H), 10.15 (s, 1H), 10.3 (br, 1H), 11.8 (brs, 1H);
Analysis: Found C,53.80; H,6.64; N,8.22; Cl,13.77; S,5.82%
C₂₃H₃₁N₃O₃S.2HCl(0.83M H₂O) requires: C,53.38; H,6.75; N,8.12; Cl,13.70; S,6.19%.

### Example 11

### 4-Hydroxy-7-[2-[6-[2-(2-pyridyl)ethoxyl]hexyl]amino-ethyl]-1,3-benzothiazol-2(3H)-one oxalate salt

A mixture of 4-Hydroxy-7-(2-aminoethyl)-1,3-benzothiazol-2(3H)-one (0.945 g), 2-(6-bromohexyloxyethyl)-pyridine (0.863 g) and diisopropylethylamine (0.66 ml) in dry dimethylformamide (25 ml) was heated at 100° for 2.5 hours. The solvent was removed under reduced pressure and the residue purified by column chromatography (SiO₂) using dichloromethane:methanol (9:1) as eluant. The resulting free base was then converted to the title oxalate salt.
mp 182-183° (decomposes);
Mass Spectrum: FAB 416 ((M+H)⁺);
nmr δ (d₆DMSO) 1.25 (m, 4H), 1.50 (m, 4H), 2.85 (m, 6H), 3.1 (m, 2H), 3.38 (m, 2H), 3.65 (m, 2H), 6.75-6.85 (dd, 2H), 7.2 (dd, 1H), 7.35 (d, 1H), 7.70 (m, 1H), 8.45 (d, 1H), 8.70 (brs, 1H), 11.75 (brs, 1H);
Analysis: Found C,53.70; H,5.98; N,7.60; S,5.56%
C₂₂H₂₉N₃O₃S.(0.5M H₂O, 0.5M excess oxalic acid) requires: C,53.54; H,5.88; N,7.48; S,5.70%.

### Example 12

### 7,7'-[1,6-Hexanediylbis(imino-2,1-ethanediyl)bis[4-hydroxy-1,3-benzothiazol-2(3H)-one difluoroacetate

### a) Bis-N,N'-[(2,4-dimethoxy-1,3-benzothiazol-7-yl)ethyl]-hexan-1,6-diamide

Adipoyl chloride (1.15 g) in dichloromethane was added dropwise over 30 min to a stirred solution of 2-(2,4-dimethoxy-1,3 -benzothiazol-7-yl)ethanamine (3.0 g, J. Med. Chem., 1987, 30, 1166) and triethylamine (3.5 ml) in dichloromethane (150 ml). The mixture was stirred at room temperature for 60 hours, filtered and the filtrate washed with dilute HCl, aqueous sodium bicarbonate and brine. The solvent was removed under reduced pressure and the residue stirred with dilute HCl. The solid was filtered, dissolved in chloroform, washed with brine, dried (MgSO₄) and the solvent removed under reduced pressure. Purification by chromatography (SiO₂) gave the sub-titled compound which was used without further purification in the next step.
Mass Spectrum: FAB 587 ((M+H)⁺).

### b) N,N'-(2-(2,4-dimethoxy-1,3-benzothiazol-7-yl)ethyl]-hex an-1,6-diamine dihydrochloride

Prepared by the method of Example 1b) using the compound from step a).

Mass Spectrum: FAB 559 ((M+H)⁺).

### c) 7,7'-[1,6-Hexanediylbis(imino-2,1-ethanediyl) bis[4hydroxy-1,3-benzothiazol-2(3H)-one difluoroacetate

The compound from step b) was dissolved in aqueous hydrobromic acid (48%) to which several drops of hypophosphorous acid had been added. The solution was refluxed for 3 hours. The solvent was removed under reduced pressure and the residue purified by preparative reverse phase HPLC to give the title compound.
mp 231-233°;
Mass Spectrum: FAB 517 ((M+H)⁺);
nmr δ (d₆DMSO) 1.32 (brs, 4H), 1.58 (brs, 4H), 2.83 (brt, 4H), 2.93 (bt, 4H), 3.10 (brt, 4H), 6.74-6.90 (m, 4H), 8.56 (brs, 2H), 10.16 (s, 2H), 11.76 (brs, 2H);
Analysis: Found C,43.11; H,4.28; N,6.78; S,8.66%
C₂₄H₃₀N₄O₄S₂ requires: C,43.11; H,3.97; N,6.77; S,7.75%.

### Example 13

### 7-[2-[1,1-Dimethyl-6-(2-phenylethoxy)hexyl]aminoethyl]-4-hydroxy-1,3-benzothiazol-2(3H)-one hydrochloride

### a) 1,1-Dimethyl-6-(2-phenylethoxy)hexanamide hydrochloride

Diphenylphosphoryl azide (6.81 g) was added to a stirred solution of 2,2-dimethyl-6-(2-phenylethoxy)hexanoic acid (6.13 g) and triethylamine (2.5 g) in toluene (100 ml) and the reaction stirred at 80° for 3 hours. The organic layer was washed with water and evaporated under reduced pressure to give a yellow oil. The residue was taken up in dioxan (100 ml), diluted with water (50 ml), and acidified to pH 1 with conc. hydrochloric acid. The solution was heated on a steam bath for 3 hours, heated under reflux for 30 min and the solvent evaporated under reduced pressure. The residue was dried by azeotropic distillation with 2-propanol and recrystallised from petroleum ether 60-80°:ethyl acetate.
mp 111-112°;
Mass Spectrum: FAB 250 ((M+H)⁺) ;
nmr δ (CDCl₃) 1.37-1.40 (brm, 4H), 1.40 (s, 6H), 1.54-1.61 (quin, 2H), 1.67-1.69 (brm, 2H), 2.85-2.89 (t, 2H), 3.39-3.42 (t, 2H), 3.58-3.62 (t, 2H), 7.17-7.30 (m, 5H), 8.33 (brs, 3H);
Analysis: Found C,67.20; H,9.86; N,5.13; Cl,12.79%
C₁₆H₂₇NO.HCl requires: C,67.22; H,9.87; N,4.90; Cl,12.40%.

### b) (2.4-Dimethoxy-1,3-benzothiazol-7-yl)acetic acid

A solution of (2,4-Dimethoxy-1,3-benzothiazol-7-yl)-acetonitrile (3.61 g, J. Med. Chem., 1987, 30, 1166) and potassium hydroxide (3.45 g) in methanol:water (2:1, 150 ml) was heated to reflux for 6.5 hours. The reaction was cooled and the solvents evaporated under reduced pressure. Water (50 ml) was added and the aqueous layer extracted with diethyl ether. The aqueous layer was then acidified to pH4/5 with acetic acid and extracted with ethyl acetate. The combined organic layers were washed (water and brine), dried (MgSO₄) and the solvent evaporated under reduced pressure to yield the sub-titled compound which was used in the next step without further purification.

### c) (4-Hydroxy-2(3H)-oxo-1,3-benzothiazol-7-yl)acetic acid

Prepared by the method of Example 14c) using the compound from step b).
mp 170-175°;
Mass Spectrum: 225 ((M+H)⁺);
nmr δ (d₆DMSO) 3.47 (s, 2H), 6.71 (d, 1H), 6.86 (d, 1H), 10.02 (brs, 1H), 11.68 (brs, 1H), 12.44 (brs, 1H);
Analysis: Found C,48.07; H,3.23; N,6.13; S,13.90%
C₉H₇NO₄S requires: C,47.99; H,3.13; N,6.22; S,14.20%.

### d) 2-(4-Hydroxy-2-oxo-3H-1,3-benzothiazol-7-yl)-N-[1,1-dimethyl-[6-(2-phenylethoxy)]hexyl]acetamide

Prepared by the method of Example la) using the compounds from steps a) and c).
Mass Spectrum: Thermal Spray Plasma 457 ((M+H)⁺);
nmr δ (CDCl₃) 1.20-1.80 (m, 8H), 1.38 (s, 6H), 2.90 (s, 2H), 3.42 (s, 2H), 3.46 (t, 2H), 3.66 (t, 2H), 5.70 (s, 1H), 6.40 (d, 1H), 6.70 (d, 1H), 7.20-7.30 (m, 5H), 8.77 (s, 1H).

### e) 7-[2-[1,1-Dimethyl-6-(2-phenylethoxy)hexyl]aminoethyl]-4-hydroxy-1.3-benzothiazol-2(3H)-one hydrochloride

Prepared by the method of Example 1b) using the compound from step d).
mp 183-185°;
Mass Spectrum: FAB 443 ((M+H)⁺);
nmr δ (d₆DMSO) 1.25 (brs, 10H), 1.45-1.60 (m, 4H), 2.79 (t, 2H), 2.90 (m, 2H), 3.00 (brs, 2H), 3.40 (m, 2H+H₂O), 3.56 (t, 2H), 6.76-6.92 (d, 2H), 7.2-7.3 (m, 5H), 8.79 (s, 2H), 10.13 (s, 1H), 11.77 (s, 1H);
Analysis: Found C,61.09; H,7.84; N,5.70; S,5.69%
C₂₅H₃₄N₂O₃S.HCl (12% ethanol) requires: C,61.40; H,7.87; N,5.11; S,5.89%.

## Claims

1. Compounds of formula I,
Ar-CH₂-CH₂-NH-CR¹R²-X-Y
in which
Ar represents a group 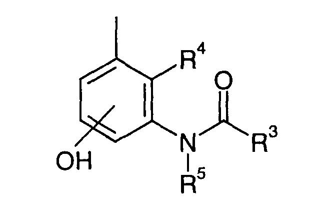
X represents a C₁₋₁₂ alkylene chain interrupted or terminated by one or more groups selected from -S-, -SO-, -SO₂-, -O-, CR⁶R⁷, phenylmethyne, -NH-, -CONH-, -NHCO- and -NHCONH-,
Y represents an aryl group of five or six atoms in a single ring system, which may contain from 1 to 3 heteroatoms selected from N, O and S, which single ring system may be optionally substituted to form a multiple fused ring system of up to 10 atoms, the aryl group being optionally substituted by one or more groups selected from -OH, halogen, C₁₋₆ alkyl, C₁₋₆ alkoxy, =O, -NH₂, or NO₂; or a C₃₋₁₂ cycloalkyl group which may contain from 1 to 3 heteroatoms selected from N, O and S, optionally substituted by one or more groups selected from -OH, halogen, C₁₋₆ alkyl, C₁₋₆ alkoxy, =O, -NH₂, or NO₂,
R¹, R², R⁵, R⁶, R⁷ and R⁸ each independently represent hydrogen or alkyl C₁₋₆, and
R³ and R⁴ represent hydrogen, or R³ and R⁴ together form a group -S-, -NR⁸- or -CH₂-,
and pharmaceutically acceptable derivatives thereof.

2. A compound of formula I, as defined in Claim 1, wherein R³ and R⁴ together represent the group -S-.

3. A compound of formula I as defined in Claim 1 or Claim 2, wherein R⁵ represents hydrogen.

4. A compound of formula I as defined in any one of the preceding claims, wherein the -OH is positioned ortho to the NH in the Ar group.

5. A compound of formula I as defined in any one of the preceding claims, wherein Y represents phenyl optionally substituted by one or more groups selected from -OH, halogen, alkyl C₁₋₆, alkoxy C₁₋₆, =O, -NH₂ or NO₂.

6. A compound of formula I which is
7-[2-[6-[2-(2,3-Dihydro- 1H-indenyloxy)]hexyl]aminoethyl]-4-hydroxy-1,3-benzthiazol-2(3H)-one,
4-Hydroxy-7-[2-[6-(2-phenylethoxy)hexyl]aminoethyl]-1,3-benzothiazol-2(3H)-one,
4-Hydroxy-7-[2-[5-(3-phenylpropoxy)pentyl]aminoethyl]-1,3-benzothiazol-2 (3H)-one,
4-Hydroxy-7-[2-[5-[2-(5-methylfuranyl)]pentyl]aminoethyl]-1,3-benzothiazol-2 (3H)-one,
4-Hydroxy-7-[2-[7-(2-phenylethoxy)heptyl]aminoethyl]-1,3-benzothiazol-2 (3H)-one,
4-Hydroxy-7-[2-[4-(2-phenylethoxy)butyl]aminoethyl]-1,3-benzothiazol-2 (3H)-one,
4-Hydroxy-7-[2-[5-(2-phenylethoxy)pentyl]aminoethyl]-1,3-benzothiazol-2 (3H)-one,
4-Hydroxy-7-[2-[6-(phenylmethoxy)hexyl]aminoethyl]-1,3-benzothiazol-2(3H)-one,
4-Hydroxy-7-[2-[4-(4-phenylbutoxy)butyl]aminoethyl]-1,3-benzothiazol-2(3H)-one,
4-Hydroxy-7-[2-(6-phenoxyhexyl)aminoethyl]-1,3-benzothiazol-2(3H)-one,
4-Hydroxy-7-[2-[6-(3-phenylpropoxy)hexyl]aminoethyl]-1,3-benzothiazol-2(3H)-one,
4-Hydroxy-7-[2-[2,2-dimethyl-6-(2-phenylethoxy)hexyl]aminoethyl]-1,3-benzothiazol-2(3H)-one,
4-Hydroxy-7-[2-[6-(2-phenylethylsulphonyl)hexyl]aminoethyl]-1,3-benzothiazol-2 (3H)-one,
N-[6-[2-(4-Hydroxy-2-oxo-3H-1,3-benzothiazol-7-yl)ethylamino]hexyl]-N'-phenylurea,
4-Hydroxy-7-[2-[6-[2-(4-nitrophenyl)ethoxy]hexyl]aminoethyl]-1,3-benzothiazol-2 (3H)-one,
4-Hydroxy-7-[2-[6-[2-(4-hydroxyphenyl)ethoxy]-2,2-dimethylhexyl]aminoethyl]-1,3-benzothiazol-2 (3H)-one,
7-Hydroxy-4-[2-[6-[2-(1-naphthyl)ethoxy]hexyl]aminoethyl]-1,3-benzothiazol-2 (3H)-one,
7-[2-[6-(2-Cyclohexylethoxy)hexyl]aminoethyl]-4-hydroxy-1,3-benzothiazol-2 (3H)-one,
7-[2-[6-[2-(4-Bromophenyl)ethoxy]hexyl]aminoethyl]-4-hydroxy-1,3-benzothiazol-2 (3H)-one,
4-Hydroxy-7-[2-[6-[2-(2-thienyl)ethoxy]hexyl]aminoethyl]-1,3-benzothiazol-2(3H)-one,
(R,S)-4-Hydroxy-7-[2-[2-methyl-6-(2-phenylethoxy)hexyl]aminoethyl]-1,3-benzothiazol-2 (3H)-one,
1-3-Dihydro-4-hydroxy-7-[2-[6-(2-phenylethoxy)hexyl]aminoethyl]-2H-benzimidazol-2-one,
4-Hydroxy-7-[2-[6-[2-(4-hydroxyphenyl)ethoxy]hexyl]aminoethyl]-1,3-benzothiazol-2 (3H)-one,
2,3-Dihydro-7-hydroxy-4-[2-[6-(phenylethoxy)hexyl]aminoethyl]-indol-2-one,
4-Hydroxy-7-[2-[3-[2-(2-phenylethyl)aminoethyl]sulphonylpropyl]aminoethyl]-1,3-benzothiazol-2(3H)-one,
(R,S)-4-Hydroxy-7-[2-[7-(2-phenylethoxy)-hept-2-yl]aminoethyl]-1,3-benzothiazol-2 (3H)-one,
6-[2-[(4-Hydroxy-2-oxo-3H-1,3-benzothiazol-7-yl)ethyl]amino]-N-(2-phenylethyl) hexanamide,
4-Hydroxy-7-[2-[6-(2-phenylethylthio)hexyl]aminoethyl]-1,3-benzothiazol-2 (3H)-one,
N-[2-Hydroxy-5-[2-N'-[6-(2-phenylethoxy)hexyl]aminoethyl]phenyl]formamide,
7-[2-[6-[2-(4-Aminophenyl)ethoxy]hexyl]aminoethyl]-4-hydroxy-1,3-benzothiazol-2 (3H)-one,
4-Hydroxy-7-[2-[6-[2-(2-pyridyl)ethoxy]hexyl]aminoethyl]-1,3-benzothiazol-2(3H)-one,
7,7'-(1,6-Hexanediylbis(imino-2,1-ethanediyl)bis[4-hydroxy-1,3-benzothiazol-2 (3H)-one],
7-[2-[1,1-Dimethyl-6-(2-phenylethoxy)hexyl]aminoethyl]-4-hydroxy-1,3-benzothiazol-2 (3H)-one,
or a pharmaceutically acceptable derivative of any one thereof.

7. A pharmaceutical composition comprising a compound of formula I, as defined in any one of the preceding claims, or a pharmaceutically acceptable derivative thereof, in association with a pharmaceutically acceptable carrier, diluent or adjuvant.

8. A method for the preparation of a compound of formula I, as defined in any one of the preceding claims, or a pharmaceutically acceptable derivative thereof, which comprises
a) alkylation of a compound of formula II, or a derivative thereof,
Ar-CH₂CH₂-NH₂ II
in which Ar is as defined in Claim 1,
with an alkylating agent of formula III,
L-CR¹R²-X-Y III
in which R¹, R², X and Y are as defined in Claim 1, and L represents a leaving group,
b) alkylation of a compound of formula II, as defined hereinabove, with a compound of formula IV,
O=CR¹-X-Y IV
in which R¹, X and Y are as defined in Claim 1, in the presence of a reducing agent,
c) selective reduction of a compound of formula V, 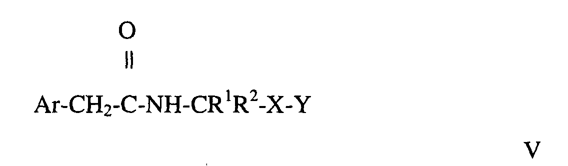 in which Ar, X, Y, R¹ and R² are as defined in Claim 1, or
for the production of compounds of formula I in which R¹ and R² represent hydrogen, selective reduction of a compound of formula Va, 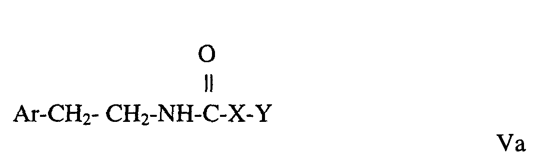 in which Ar, X and Y are as defined in Claim 1,
d) removal of a protecting group from a corresponding protected compound of formula I in which one or more of the functional groups is protected,
and where desired or necessary converting a compound of formula I to a pharmaceutically acceptable derivative thereof or vice versa.

9. Compounds of formula Va, 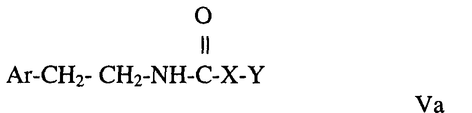 in which Ar, X and Y are as defined in Claim 1.

## Patentansprüche

1. Verbindungen der Formel I
Ar-CH₂-CH₂-NH-CR¹R²*-*X*-*Y,
in welcher
Ar für eine Gruppe 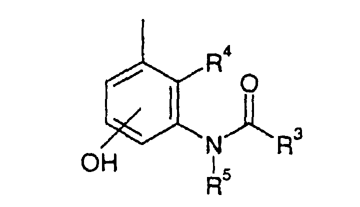 steht,
X für eine C₁₋₁₂-Alkylenkette steht, die durch eine oder mehrere Gruppen ausgewählt aus -S-, -SO-, -SO₂-, -O-, CR⁶R⁷, Phenylmethin, -NH-, -CONH-, -NHCO-und -NHCONH- unterbrochen oder terminiert ist,
Y für eine Arylgruppe von fünf oder sechs Atomen in einem Einzelringsystem steht, welches 1 bis 3 Heteroatome ausgewählt aus N, O und S enthalten kann, wobei das Einzelringsystem gegebenenfalls so substituiert sein kann, daß es ein kondensiertes Mehrfachringsystem von bis zu 10 Atomen bildet, wobei die Arylgruppe gegebenenfalls durch eine oder mehrere Gruppen ausgewählt aus -OH, Halogen, C₁₋₆-Alkyl, C₁₋₆-Alkoxy, =O, -NH₂ oder NO₂ substituiert ist; oder für eine C₃₋₁₂-Cycloalkylgruppe, die 1 bis 3 Heteroatome ausgewählt aus N, O und S enthalten kann und gegebenenfalls durch eine oder mehrere Gruppen ausgewählt unter -OH, Halogen, C₁₋₆-Alkyl, C₁₋₆-Alkoxy, =O, -NH₂ oder NO₂ substituiert ist,
R¹, R², R⁵, R⁶, R⁷ und R⁸ jeweils unabhängig für Wasserstoff oder C₁₋₆-Alkyl stehen, und
R³ und R⁴ für Wasserstoff stehen, oder R³ und R⁴ zusammen eine Gruppe -S-, -NR⁸- oder -CH₂- bilden,
und deren pharmazeutisch verträgliche Derivate.

2. Verbindungen der Formel I nach Anspruch 1, wobei R³ und R⁴ gemeinsam für die Gruppe -S- stehen.

3. Verbindungen der Formel I nach Anspruch 1 oder Anspruch 2, wobei R⁵ für Wasserstoff steht.

4. Verbindungen der Formel I nach einem der vorhergehenden Ansprüche, wobei das -OH sich in der ortho-Stellung zum NH in der Ar-Gruppe befindet.

5. Verbindungen der Formel I nach einem der vorhergehenden Ansprüche, wobei Y für Phenyl steht, welches gegebenenfalls durch ein oder mehrere Gruppen ausgewählt aus -OH, Halogen, C₁₋₆-Alkyl, C₁₋₆-Alkoxy, =O, -NH₂ oder NO₂ substituiert ist.

6. Verbindungen der Formel I, bei denen es sich um 7-[2-[6-[2-(2,3-Dihydro-lH-indenyloxy)]hexyl]-aminoethyl]-4-hydroxy-1,3-benzothiazol-2(3H)-on,
4-Hydroxy-7-[2-[6-(2-phenylethoxy)hexyl]aminoethyl]-1,3-benzothiazol-2(3H)-on,
4-Hydroxy-7-[2-[5-(3-phenylpropoxy)pentyl]aminoethyl]-1,3-benzothiazol-2(3H)-on,
4-Hydroxy-7-[2-[5-[2-(5-methylfuranyl)]pentyl]-aminoethyl]-1,3-benzothiazol-2(3H)-on,
4-Hydroxy-7-[2-[7-(2-phenylethoxy)heptyl]aminoethyl]-1,3-benzothiazol-2(3H)-on,
4-Hydroxy-7-[2-[4-(2-phenylethoxy)butyl]aminoethyl]-1,3-benzothiazol-2(3H)-on,
4-Hydroxy-7-[2-[5-(2-phenylethoxy)pentyl]aminoethyl]-1,3-benzothiazol-2(3H)-on,
4-Hydroxy-7-[2-[6-(phenylmethoxy)hexyl]aminoethyl]-1,3-benzothiazol-2(3H)-on,
4-Hydroxy-7-[2-[4-(4-phenylbutoxy)butyl]aminoethyl]-1,3-benzothiazol-2(3H)-on,
4-Hydroxy-7-[2-[6-(phenoxyhexyl)aminoethyl]-1,3-benzothiazol-2(3H)-on,
4-Hydroxy-7-[2-[6-(3-phenylpropoxy)hexyl]aminoethyl]-1,3-benzothiazol-2(3H)-on,
4-Hydroxy-7-[2-[2,2-dimethyl-6-(2-phenylethoxy)hexyl]-aminoethyl]-1,3-benzothiazol-2(3H)-on,
4-Hydroxy-7-[2-[6-(2-phenylethylsulfonyl)hexyl]-aminoethyl]-1,3-benzothiazol-2(3H)-on,
N-[6-[2-(4-Hydroxy-2-oxo-3H-1,3-benzothiazol-7-yl)-ethylamino]hexyl]-N'-phenylharnstoff,
4-Hydroxy-7-[2-[6-[2-(4-nitrophenyl)ethoxy]hexyl]-aminoethyl]-1,3-benzothiazol-2(3H)-on,
4-Hydroxy-7-[2-[6-[2-(4-hydroxyphenyl)ethoxy]-2,2-dimethylhexyl]aminoethyl]-1,3-benzothiazol-2(3H)-on,
7-Hydroxy-4-[2-[6-[2-(1-naphtyl)ethoxy]hexyl]-aminoethyl]-1,3-benzothiazol-2(3H)-on,
7-[2-[6-(2-Cyclohexylethoxy)hexyl]aminoethyl]-4-hydroxy-1,3-benzothiazol-2(3H)-on,
7-[2-[6-[2-(4-Bromphenyl)ethoxy]hexyl]aminoethyl]-4-hydroxy-1,3-benzothiazol-2(3H)-on,
4-Hydroxy-7-[2-[6-[2-(2-thienyl)ethoxy])hexyl]-aminoethyl]-1,3-benzothiazol-2(3H)-on,
(R,S) -4-Hydroxy-7-[2-[2-methyl-6-(2-phenylethoxy)-hexyl]aminoethyl]-1,3-benzothiazol-2(3H)-on,
1-3-Dihydro-4-hydroxy-7-[2-[6-(2-phenylethoxy)-hexyl]aminoethyl]-2H-benzimidazol-2-on,
4-Hydroxy-7-[2-[6-[2-(4-hydroxyphenyl)ethoxy])hexyl]-aminoethyl]-1,3-benzothiazol-2(3H)-on,
2,3-Dihydro-7-hydroxy-4-[2-[6-(phenylethoxy)hexyl]-aminoethyl]-indol-2-on,
4-Hydroxy-7-[2-[3-[2-(2-phenylethyl)aminoethyl]-sulfonylpropyl]aminoethyl]-1,3-benzothiazol-2(3H)-on,
(R,S)-4-Hydroxy-7-[2-[7-(2-phenylethoxy)-hept-2-yl]-aminoethyl]-1,3-benzothiazol-2(3H)-on,
6-[2-[(4-Hydroxy-2-oxo-3H-1,3-benzothiazol-7-yl)-ethyl]amino]-N-(2-phenylethyl)hexanamid,
4-Hydroxy-7-[2-[6-(2-phenylethylthio)hexyl]aminoethyl]-1,3-benzothiazol-2(3H)-on,
N-[2-Hydroxy-5-[2-N'-[6-(2-phenylethoxy)hexyl]-aminoethyl]phenyl]formamid,
7-[2-[6-[2-(4-Aminophenyl)ethoxy]hexyl]aminoethyl]-4-hydroxy-1,3-benzothiazol-2(3H)-on,
4-Hydroxy-7-[2-[6-[2-(2-pyridyl)ethoxy]hexyl]-aminoethyl)-1,3-benzothiazol-2(3H)-on,
7,7'-(1,6-Hexandiylbis(imino-2,1-ethandiyl)bis[4-hydroxy-1,3-benzothiazol-2(3H)-on],
7-[2-[1,1-Dimethyl-6-(2-phenylethoxy)hexyl]aminoethyl]-4-hydroxy-1,3-benzothiazol-2(3H) -on,
oder ein pharmazeutisch verträgliches Derivat einer dieser Verbindungen handelt.

7. Pharmazeutische Zusammensetzung, enthaltend eine Verbindung der Formel I nach einem der vorhergehenden Ansprüche oder ein pharmazeutisch verträgliches Derivat davon in Verbindung mit einem pharmazeutisch verträglichen Trägerstoff, Verdünnungsmittel oder Zusatzstoff.

8. Methode zur Herstellung einer Verbindung der Formel I nach einem der vorhergehenden Ansprüche oder eines pharmazeutisch verträglichen Derivats davon, umfassend
a) Alkylierung einer Verbindung der Formel II oder eines Derivats davon
Ar-CH₂CH₂-NH₂ II
in welcher Ar wie in Anspruch 1 definiert ist,
mit einem Alkylierungsmittel der Formel III
L-CR¹R²-X-Y III
in welcher R¹, R², X und Y wie in Anspruch 1 definiert sind und L für eine Abgangsgruppe steht,
b) Alkylierung einer wie oben definierten Verbindung der Formel II mit einer Verbindung der Formel IV
O=CR¹-X-Y IV
in welcher R¹, X und Y wie in Anspruch 1 definiert sind, in Gegenwart eines Reduktionsmittels,
c) selektive Reduktion einer Verbindung der Formel V 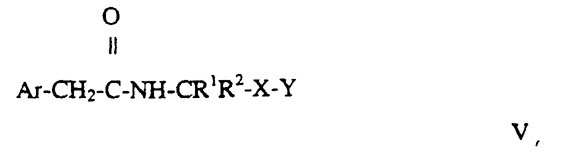 in welcher Ar, X, Y, R¹ und R² wie in Anspruch 1 definiert sind, oder,
zur Herstellung von Verbindungen der Formel I, in welcher R¹ und R² für Wasserstoff stehen,
selektive Reduktion einer Verbindung der Formel Va 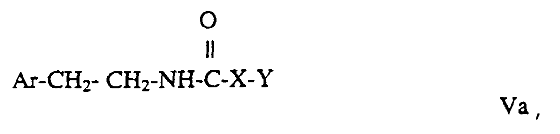 in welcher Ar, X und Y wie in Anspruch 1 definiert sind,
d) Entfernung einer Schutzgruppe aus einer entsprechenden geschützten Verbindung der Formel I, in welcher ein oder mehrere der funktionellen Gruppen geschützt sind,
und, falls gewünscht oder erforderlich, Umwandlung einer Verbindung der Formel I in ein pharmazeutisch verträgliches Derivat davon, oder umgekehrt.

9. Verbindungen der Formel Va 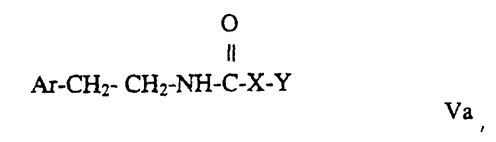 in welcher Ar, X und Y wie in Anspruch 1 definiert sind.

## Revendications

1. Composés de formule I,
Ar-CH₂-CH₂-NH-CR¹R²-X-Y
dans lesquels
Ar représente un groupement 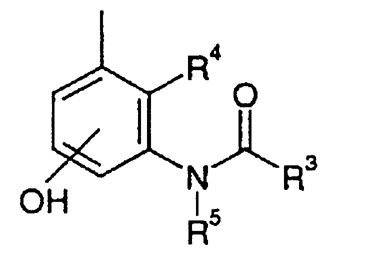
X représente une chaîne alkylène en C₁-C₁₂ interrompue ou terminée par un ou plusieurs groupements sélectionnés parmi -S-, -SO-, -SO₂-, -O-, CR⁶R⁷, un phénylméthyne, -NH-, -CONH-, -NHCO- et -NHCONH-,
Y représente un groupement aryle de cinq ou six atomes en un unique système cyclique, qui peut contenir de 1 à 3 hétéroatomes sélectionnés parmi N, O et S, lequel unique système cyclique peut être facultativement substitué pour former un système cyclique multiple fusionné de jusqu'à 10 atomes, le groupement aryle étant facultativement substitué par un ou plusieurs groupements sélectionnés parmi -OH, un halogène, un alkyle en C₁-C₆, un alcoxy en C₁-C₆, =O, -NH₂, ou NO₂; ou un groupement cycloalkyle en C₃-C₁₂ qui peut contenir de 1 à 3 hétéroatomes sélectionnés parmi N, O et S, facultativement substitué par un ou plusieurs groupements sélectionnés parmi -OH, un halogène, un alkyle en C₁-C₆, un alcoxy en C₁-C₆, =O, -NH₂, ou NO₂,
R¹, R², R⁵, R⁶, R⁷ et R⁸ représentent chacun indépendamment un hydrogène ou un alkyle en C₁-C₆, et
R³ et R⁴ représentent un hydrogène, ou R³ et R⁴ forment ensemble un groupement -S-, -NR⁸- ou -CH₂-,
et des dérivés pharmaceutiquement acceptables de ceux-ci.

2. Composé de formule I, comme défini dans la revendication 1, dans lequel R³ et R⁴ ensemble représentent le groupement -S-.

3. Composé de formule I, comme défini dans la revendication 1 ou dans la revendication 2, dans lequel R⁵ représente un hydrogène.

4. Composé de formule I comme défini dans l'une quelconque des revendications précédentes, dans lequel le -OH est positionné en ortho du NH dans le groupement Ar.

5. Composé de formule I comme défini dans l'une quelconque des revendications précédentes, dans lequel Y représente un phényle facultativement substitué par un ou plusieurs groupements sélectionnés parmi -OH, un halogène, un alkyle en C₁-C₆, un alcoxy en C₁-C₆, =O, -NH₂, ou NO₂.

6. Composé de formule I qui est
la 7-[2-[6-[2-(2,3-dihydro-lH-indényloxy)]hexyl]-aminoéthyl]-4-hydroxy-1,3-benzothiazol-2-(3H)-one,
la 4-hydroxy-7-[2-[6-(2-phényléthoxy)hexyl]amino-éthyl]-1,3-benzothiazol-2-(3H)-one,
la 4-hydroxy-7-[2-[5-(3-phénylpropoxy)pentyl]-aminoéthyl]-1,3-benzothiazol-2-(3H)-one,
la 4-hydroxy-7-[2-[5-[2-(5-méthylfuranyl)]-pentyl]aminoéthyl]-1,3-benzothiazol-2-(3H)-one,
la 4-hydroxy-7-[2-[7-(2-phényléthoxy)heptyl]amino-éthyl]-1,3-benzothiazol-2-(3H)-one,
la 4-hydroxy-7-[2-[4-(2-phényléthoxy)butyl]amino-éthyl]-1,3-benzothiazol-2-(3H)-one,
la 4-hydroxy-7-[2-[5-(2-phényléthoxy)pentyl]amino-éthyl]-1,3-benzothiazol-2-(3H)-one,
la 4-hydroxy-7-[2-[6-(phénylméthoxy)hexyl]amino-éthyl]-1,3-benzothiazol-2-(3H)-one,
la 4-hydroxy-7-[2-[4-(4-phénylbutoxy)butyl]amino-éthyl]-1,3-benzothiazol-2-(3H)-one,
la 4-hydroxy-7-[2-(6-phénoxyhexyl)aminoéthyl]-1,3-benzothiazol-2-(3H)-one,
la 4-hydroxy-7-[2-[6-(3-phénylpropoxy)hexyl]amino-éthyl]-1,3-benzothiazol-2-(3H)-one,
la 4-hydroxy-7-[2-[2,2-diméthyl-6-(2-phényl-éthoxy)hexyl]aminoéthyl]-1,3-benzothiazol-2-(3H)-one,
la 4-hydroxy-7-[2-[6-(2-phényléthylsulfonyl)-hexyl]aminoéthyl]-1,3-benzothiazol-2-(3H)-one,
la N-[6-[2-(4-hydroxy-2-oxo-3H-1,3-benzothiazol-7-yl)éthylamino]hexyl]-N'-phénylurée,
la 4-hydroxy-7-[2-[6-[2-(4-nitrophényl)éthoxy]-hexyl]aminoéthyl]-1,3-benzothiazol-2-(3H)-one,
la 4-hydroxy-7-[2-[6-[2-(4-hydroxyphényl)éthoxy]-2,2-diméthylhexyl]aminoéthyl]-1,3-benzothiazol-2-(3H)-one,
la 7-hydroxy-4-[2-[6-[2-(1-naphtyl)éthoxy]-hexyl]aminoéthyl]-1,3-benzothiazol-2-(3H)-one,
la 7-[2-[6-(2-cyclohexyléthoxy)hexyl]aminoéthyl]-4-hydroxy-1,3-benzothiazol-2-(3H)-one,
la 7-[2-[6-[2-(4-bromophényl)éthoxy]hexyl]amino-éthyl]-4-hydroxy-1,3-benzothiazol-2-(3H)-one,
la 4-hydroxy-7-[2-[6-[2-(2-thiényl)éthoxy]hexyl]-aminoéthyl]-1,3-benzothiazol-2-(3H)-one,
les (R,S)-4-hydroxy-7-[2-[2-méthyl-6-(2-phényl-éthoxy)hexyl]aminoéthyl]-1,3-benzothiazol-2-(3H)-one,
la 1,3-dihydro-4-hydroxy-7-[2-[6-(2-phényl-éthoxy)hexyl]aminoéthyl]-2H-benzimidazol-2-one,
la 4-hydroxy-7-[2-[6-[2-(4-hydroxyphényl)éthoxy]-hexyl]aminoéthyl]-1,3-benzothiazol-2-(3H)-one,
la 2,3-dihydro-7-hydroxy-4-[2-[6-(phényléthoxy)-hexyl]aminoéthyl]-indol-2-one,
la 4-hydroxy-7-[2-[3-[2-(2-phényléthyl)amino-éthyl]sulfonylpropyl]aminoéthyl]-1,3-benzothiazol-2-(3H)-one,
les (R,S)-4-hydroxy-7-[2-[7-(2-phényléthoxy)-hept-2-yl]aminoéthyl]-1,3-benzothiazol-2-(3H)-one,
le 6-[2-[(4-hydroxy-2-oxo-3H-1,3-benzothiazol-7-yl)éthyl]amino]-N-(2-phényléthyl)hexanamide,
la 4-hydroxy-7-[2-[6-(2-phényléthylthio)hexyl]-aminoéthyl]-1,3-benzothiazol-2-(3H)-one,
le N-[2-hydroxy-5-[2-N'-[6-(2-phényléthoxy)hexyl]-aminoéthyl]phényl]formamide,
la 7-[2-[6-[2-(4-aminophényl)éthoxy]hexyl]amino-éthyl]-4-hydroxy-1,3-benzothiazol-2-(3H)-one,
la 4-hydroxy-7-[2-[6-[2-(2-pyridyl)éthoxy]hexyl]-aminoéthyl]-1,3-benzothiazol-2-(3H)-one],
la 7,7'-(1,6-hexanediylbis(imino-2,1-éthanediyl)-bis[4-hydroxy-1,3-benzothiazol-2-(3H)-one,
la 7-[2-[1,1-diméthyl-6-(2-phényléthoxy)hexyl]-aminoéthyl]-4-hydroxy-1,3-benzothiazol-2-(3H)-one,
ou un dérivé pharmaceutiquement acceptable d'un quelconque de ceux-ci.

7. Composition pharmaceutique comprenant un composé de formule I, comme défini dans l'une quelconque des revendications précédentes, ou un dérivé pharmaceutiquement acceptable de celui-ci, en association avec un vecteur, un diluant ou un adjuvant pharmaceutiquement acceptable.

8. Procédé de préparation d'un composé de formule I, comme défini dans l'une quelconque des revendications précédentes, ou d'un dérivé pharmaceutiquement acceptable de celui-ci, qui comprend
a) l'alkylation d'un composé de formule II, ou d'un dérivé de celui-ci,
Ar-CH₂-CH₂-NH₂ II
dans lequel Ar est comme défini à la revendication 1,
avec un agent alkylant de formule III,
L-CR¹R²-X-Y III
dans lequel R¹, R², X et Y sont comme définis dans la revendication 1, et L représente un groupement sortant,
b) l'alkylation d'un composé de formule II, comme défini ci-dessus dans le présent mémoire, avec un composé de formule IV,
O=CR¹-X-Y IV
dans lequel R¹, X et Y sont comme définis à la revendication 1, en présence d'un agent réducteur,
c) la réduction sélective d'un composé de formule V, 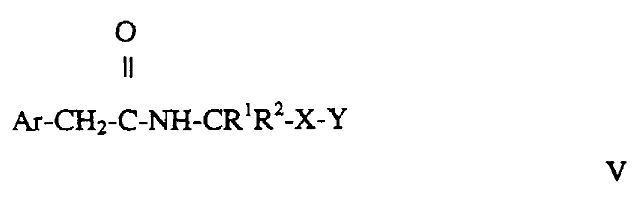 dans lequel Ar, X, Y, R¹ et R² sont comme définis dans la revendication 1, ou
pour la production de composés de formule I dans lesquels R¹ et R² représentent un hydrogène, la réduction sélective d'un composé de formule Va, 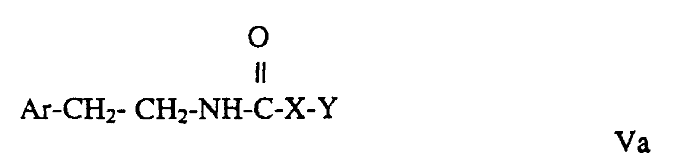 dans lequel Ar, X et Y sont comme définis dans la revendication 1,
d) l'enlèvement d'un groupement protecteur à partir d'un composé protégé correspondant de formule I dans lequel un ou plusieurs des groupements fonctionnels sont protégés,
et lorsque souhaité ou nécessaire la conversion d'un composé de formule I en un dérivé pharmaceutiquement acceptable de celui-ci ou inversement.

9. Composés de formule Va 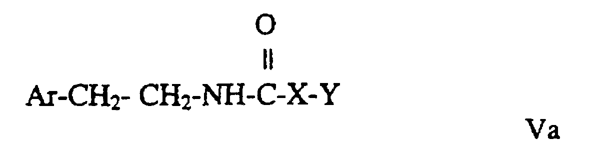 dans lesquels Ar, X et Y sont comme définis dans la revendication 1.
